(19) **Europäisches Patentamt**
**European Patent Office**
**Office européen des brevets**

(11) **EP 3 074 758 B1**

(12) **EUROPEAN PATENT SPECIFICATION**

(45) Date of publication and mention
of the grant of the patent:
**27.02.2019 Bulletin 2019/09**

(51) Int Cl.:
*G01N 21/78* (2006.01)          *G01N 21/84* (2006.01)
*G01J 3/433* (2006.01)          *G01N 33/487* (2006.01)
*G01N 21/27* (2006.01)          *G01N 21/47* (2006.01)
*G01N 21/77* (2006.01)

(21) Application number: **14808560.8**

(22) Date of filing: **27.11.2014**

(86) International application number:
**PCT/EP2014/075780**

(87) International publication number:
**WO 2015/078954 (04.06.2015 Gazette 2015/22)**

(54) **METHOD AND DEVICE FOR DETERMINING A CONCENTRATION OF AN ANALYTE IN A BODILY FLUID**

VERFAHREN UND VORRICHTUNG ZUR BESTIMMUNG DER KONZENTRATION EINES ANALYTS IN EINER KÖRPERFLÜSSIGKEIT

PROCÉDÉ ET DISPOSITIF POUR DÉTERMINER UNE CONCENTRATION D'UN ANALYTE DANS UN FLUIDE CORPOREL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2013 EP 13194791**

(43) Date of publication of application:
**05.10.2016 Bulletin 2016/40**

(73) Proprietors:
• **Roche Diabetes Care GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**

(72) Inventors:
• **ALBRECHT, Gertrud**
  **68239 Mannheim (DE)**
• **BAUMANN, Edgar**
  **68219 Mannheim (DE)**
• **GENTHNER-RIEGLER, Markus**
  **69121 Heidelberg (DE)**
• **KALVERAM, Stefan**
  **68519 Viernheim (DE)**
• **NIESPOREK, Christian**
  **69120 Heidelberg (DE)**
• **SCHWENKER, Kai-Oliver**
  **67454 Haßloch (DE)**
• **SERR, Markus**
  **CH-6300 Zug (CH)**
• **WEHOWSKI, Frédéric**
  **68766 Hockenheim (DE)**
• **WETTENGEL, Klaus**
  **67592 Flörsheim-Dalsheim (DE)**

(74) Representative: **Herzog, Fiesser & Partner**
**Patentanwälte PartG mbB**
**Dudenstrasse 46**
**68167 Mannheim (DE)**

(56) References cited:
**EP-B1- 1 912 058      US-A- 5 526 120**
**US-A- 6 055 060      US-A1- 2003 169 426**

## Description

FIELD OF THE INVENTION

The present invention discloses a method, an analytical device and an analytical system for determining a concentration of at least one analyte in a bodily fluid. The method, system and use according to the present invention may specifically be used for determining the concentration of glucose in one or more bodily fluids, such as in whole blood. Additionally or alternatively, however, one or more other types of analytes and/or one or more other types of bodily fluids may be used. The invention may be applied in the field of diabetes care, both in home monitoring and in hospital applications. Additionally or alternatively, other uses are feasible.

BACKGROUND OF THE INVENTION

In the art, a large number of device methods for determining the concentration of one or more analytes in bodily fluids are known. Without restricting the scope of the present invention, in the following, mainly reference is made to the determination of glucose as an exemplary and preferred analyte.

The determination of the concentration of one or more analytes in a bodily fluid specifically may be performed by using photometric measurements. A sample of the bodily fluid may be applied onto a test carrier, which is illuminated by light to perform the photometric measurement. Typically, reflective measurements are performed, in order to determine an amount of light elastically or inelastically reflected, scattered or remitted by the test carrier. The test carrier typically is based on the use of at least one test chemical, i.e. on the use of one or more chemical compounds or chemical mixtures adapted for performing a detectable reaction, which yields to a detectable change of the test carrier, in particular to an optical change such as a color change. The test chemical often is also referred to a test substance, a test chemistry, a test reagent or a detector substance. For details of potential test chemicals and test elements comprising such test chemicals, which may also be used within the present invention, reference can be made to J. Hones et al.: "The Technology Behind Glucose Meters: Test Strips", Diabetes Technology & Therapeutics, Vol. 10, supplement 1, 2008, S-10 to S-26. Other types of test elements and/or test substances are feasible and may be used within the present invention.

By using one or more test chemicals, a detection reaction may be initiated, the course of which depends on the concentration of the analyte to be determined. Typically, as may also be the case in the present invention, the test chemical is adapted to perform at least one detection reaction when the analyte is present in the bodily fluid, wherein the extent and/or degree of the detection reaction, such as the kinetics of the detection reaction, depends on the concentration of the analyte. In case of photometric measurements, the test carrier may be illuminated with light, wherein the light may be diffusely reflected from the test carrier and detected by an analyzing device. For example, the concentration of the analyte in a sample can be determined by measuring the reflectivity of the test carrier, when the detection reaction is completed. Additionally or alternatively the progress of the detection reaction may be monitored by measuring a temporal change of the reflectivity. Thus in photometric measurements, the test chemical preferably may be adapted to change at least one reflective property due to the detection reaction, preferably a color.

The measurement and the analysis of the remitted light typically impose some technical challenges. On the one hand, these measurements typically involve small currents and/or voltages. A measurement of such small currents or voltage, however, is challenging since interferences may occur, such as interferences due to low-frequency voltages. On the other hand optical disturbances may occur because of ambient light. Thus for determination of the concentration of an analyte in the sample with photometric measurements analyzing devices and methods are needed to reduce inference of these disturbances.

In EP 0 632 262 B1 a method for detecting and evaluating analog photometric signals in a test carrier analysis apparatus is proposed. The test field of the test carrier is irradiated by a light source which is clocked in light and dark phases. The light and dark phases form an irregular sequence with a frequency spectrum having a large number of different frequencies. The light is reflected and detected by a measurement receiver and its measured value is passed to a measurement integration and digitalization circuit for evaluation, where the irregular signal is filtered out.

In EP 1 912 058 B1 a system adapted for measuring and evaluating optical signals for detecting an analyte in an analysis liquid is described. The system comprises a test carrier and a light source for illuminating an optical evaluation zone of the test carrier. In addition, the system comprises two signal sources adapted for generating two control signals, mixed by a mixer unit to generate a light control signal for the light source. A light sensor receives the remitted light and converts it into a measuring signal. Further the system comprises two frequency-selective amplifiers, each receiving the measurement signal and one of the control signals, and an evaluation unit, to which the output signal of the frequency-selective amplifiers are fed. In the evaluation unit these output signals are compared and information about interference of the measurement by external light is determined from the result of the comparison. In case an interference of the measurement above a certain threshold is determined a measurement is recognized as faulty. The measurement is rejected and no glucose concentration value is issued.

Further, in many cases, the test carrier has to be oriented within the device for determining the concentration of one or more analytes such that the device is able to perform a determination of the concentration. In US 2003/169426 A1 a test meter capable of determining the orientation of a test member within it is described. The test member has a first major surface and an opposing second major surface. Each major surface includes an orientation indicator region, the orientation indicator regions differing by at least one optical property, for example reflectance. The test meter has a test region for accepting a test member and comprising an optical orientation sensor. The optical orientation sensor generates an orientation signal indicative of an optical property of the orientation indicator region.

In US 5 526 120 A a test strip and apparatus each having an asymmetry are proposed. The asymmetries combine to permit a test strip to be inserted into the apparatus when it is correctly aligned but prevent the test strip from being fully inserted if it is wrong side up. The apparatus detects whether or not the strip has been fully inserted.

Despite the advantages implied by these devices and methods known in the art, still, a large number of technical challenges remains. Thus, as an example, many devices and methods known in the art are not suited for recognizing disturbances before or while measuring. These disturbances, as an example, may arise from internal disturbances such as fluctuations of one or more light sources and/or noise within electronic components of the devices. Further, external disturbances have to be considered, such as disturbances induced by ambient light. These disturbances may lead to significant faults and falsifications of the measurement results. Typical methods and devices known in the art, however, allow for a fault detection at the end of each measurement, only. For example, in paragraph [0047] of EP 1 912 058 B1 it is disclosed to compare analytical results which have been determined from raw data of output signals of a frequency-selective amplifier, and not raw data directly. Thus, in case a measurement is rejected, the whole test carrier wetted by the sample is rejected and a new sample has to be applied on a new test carrier, implying that the new sample has to be taken from a patient or user. Thus, generally, known methods and devices typically imply the drawback that test carriers are wasted and that the user or patient, at least to some extent, will have to generate a sample of the bodily fluid repeatedly in order to obtain a reliable measurement. Further, specifically in view of an increased use of modern light sources like energy-saving lamps, LEDs etc., and in view of an increased trend towards miniaturization of analytical devices, disturbances of photometric measurements may increase. Consequently, a strong need exists for methods and devices which are suited to at least partially avoid waste of test carriers and frequent generation of samples, by still providing fast and reliable measurement results.

Further, EP 1 912 058 B1 discloses that a first signal source generates a first control signal with a base frequency and a second signal source generates a second control signal with a frequency which is a multiple of the base frequency. The intensities of the first control signal and the second control signal are different from each other. However, using of control signals with different intensities may enhance the possibility of a faulty fault detection in case of low measurement signals, because of faultily identifying a low measurement signal and not detecting a disturbance. Thus, even though a valid measurement signal was measured, the valid measurement signal may erroneously be identified as a disturbance rather than as a valid measurement signal.

It is therefore an objective of the present invention to provide methods and devices for determining the concentration of an analyte in a bodily fluid which overcome the above-mentioned shortcomings and challenges of known methods and devices. Preferably, methods and devices shall be disclosed which are capable of reliably determining a concentration of an analyte in a bodily fluid even in the presence of disturbances.

## SUMMARY OF THE INVENTION

This problem is solved by a method, as defined in claim 1, an analytical device, as defined in claim 16, and an analytical system, as defined in claim 17, for determining a concentration of at least one analyte in a bodily fluid. Particular embodiments, which might be realized in an isolated fashion or in any arbitrary combination, are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

In a first aspect of the present invention, a method for determining a concentration of at least one analyte in a bodily fluid comprising the features of appended claim 1 is disclosed.

The bodily fluid generally may be or may be selected from an arbitrary type of bodily fluid, preferably from the group consisting of: blood, preferably whole blood; interstitial fluid; urine; saliva. Additionally or alternatively, other types of bodily fluids may be used. Additionally or alternatively, also further processed bodily fluids like blood plasma or blood serum may be used.

The analyte generally may be a substance or compound or a combination of substances or compounds which may be present in the body fluid. The analyte may be a substance which is part of a metabolism of a human or animal being or which may take part in the metabolism. Specifically, the analyte may be a metabolite. Preferably, the analyte is selected from the group consisting of: glucose, lactate, triglycerides, ketone, ethanol, total cholesterol, HDL cholesterol, LDL cholesterol, urea, uric acid, creatinine, GOT, GPT, GGT, ammonia. Additionally or alternatively, also other clinical chemical parameters or analytes like alkaline phosphatase (ALP), creatine kinase (CK), amylaea, pancraetic amylase, (Gamma)-Glutamyltransferase (GGT), Glutamic-oxaloacetic transaminase (GOT), Glutamic-pyruvic transaminase (GPT), bilirubin, hemoglobin, potassium. Additionally or alternatively, the analytes may be substances or combination of substances involved in the intrinsic and/or extrinsic coagulation pathway. Generally, the analyte may be any type of clinical parameter of the bodily fluid that might be of interest for clinical purposes, such as any type of clinical parameter that might be determined from whole blood. Without restricting further embodiments of the present invention, in the following, in most parts reference will be made to the detection of glucose in whole blood.

The method comprises the method steps as given in claim 1 and as listed as follows. The method steps may be performed in the given order, i.e. in the order a) - b) - c) - d). However, other orders of the method steps are feasible, such as b) - a) - c) - d). Further, one or more of the method steps may be performed in parallel and/or in a timely overlapping fashion, such as by performing method steps a) and b) at least partially simultaneously and/or by performing method steps b), c) and d) at least partially simultaneously. Further, one or more of the method steps may be performed repeatedly. Thus, as an example, method steps b) and/or c) may be performed repeatedly, such as by performing method steps b) and/or c) at least once before method step a) and performing method steps b) and/or c) at least once after performing method step a). Further, additional method steps may be present which are not listed.

The method steps are as follows:

a) applying a sample of the bodily fluid to a test carrier;
b) illuminating the test carrier by using at least one light source;
c) receiving light remitted by the test carrier by using at least one detector;
d) determining the concentration of the analyte by evaluating at least one detector signal generated by the detector.

Therein, the at least one light source is modulated by using at least two different modulation frequencies. The detector signal is demodulated with the at least two different modulation frequencies in order to generate at least two demodulated detector signals, each demodulated detector signal corresponding to one of the modulation frequencies. The method further comprises a fault detection based on a comparison of the at least two demodulated detector signals. The at least one light source comprises at least one first light source being modulated by at least two different modulation frequencies and at least one second light source being modulated by at least two different modulation frequencies being different from the at least two different modulation frequencies by which the first light source is modulated.

As used herein, an application of a sample of a bodily fluid to a test carrier generally refers to the step of bringing the test carrier in contact with the sample of the bodily fluid in any technically feasible way. The application may take place manually or automatically, such as by applying the sample to at least one application position. The sample may be applied to a test chemical of the test carrier, such as a test field comprising the at least one test chemical. Additionally or alternatively, the sample may be applied to a different application position, such as to an opening of a capillary element adapted to transport the sample to the test chemical by capillary forces. The application of the sample of the bodily fluid to the test carrier may take place before, during or after insertion of the test carrier into a receptacle of an analytical device adapted for performing the method. Generally, means and devices for applying the sample to the test carrier are known to the skilled person.

In general, further method steps may be performed, such as before method steps a) - d), for example before the application of a sample of bodily fluid to a test carrier as proposed in method step a). Further, in certain embodiments, further method steps may be performed even without using a test carrier. Thus, as outlined in further detail below, it may be possible to perform an ambient light fault detection step and/or a determination of a dry empty value and/or a position verification step, before performing method steps a) - d). Thus, in case the optional ambient light fault detection step should reveal that the ambient light does not allow for an analyte measurement (such as in case an ambient light level, at least at certain modulation frequencies, should be above a tolerance threshold), the measurement may be aborted, without subsequently performing a sample application. Similarly, additionally or alternatively, in case the determination of at least one dry empty value, should come to the conclusion that the test carrier is unusable, such as due to aging or deterioration effects, the measurement may be aborted, without subsequently performing a sample application. Similarly, additionally or alternatively, in case the position verification step should reveal that the test carrier is misplaced or not aligned properly, the measurement may be aborted, without subsequently performing a sample application.

As used herein, the term test carrier generally refers to a test element adapted for determining the concentration of the at least one analyte in the bodily fluid. Specifically, the test carrier may be an optical test carrier adapted for optically determining the concentration of the analyte. The test carrier generally may have any technically feasible format. The test carrier may comprise one or more test chemicals which directly or indirectly may be contacted with the bodily fluid. For potential embodiments of the at least one test chemical, reference may be made to the disclosure of potential test chemicals given above or given in further detail below. Specifically, the test carrier may comprise one or more test fields having one or more continuous or discontinuous detection layers comprising the at least one test chemical. Additionally, one or more additional layers may be present, such as one or more reflective layers having one or more colored pigments such as white pigments and/or one or more separation layers adapted for separating off one or more components of the body fluid such as one or more cellular components. Other embodiments are feasible. The test carrier generally may have an arbitrary form or format, such as one or more of the test carrier formats known in the art. As an example, the test carrier may be selected from the group consisting of a test strip, a test tape, a test disc, and an integrated test carrier having at least one test chemical and at least one lancet element.

The test carrier may comprise at least one substrate and at least one test chemical directly or indirectly applied to the substrate. The test chemical may be adapted to perform at least one detection reaction in the presence of the at least one analyte to be detected and to change at least one optically detectable property due to the detection reaction. For potential embodiments of the test chemical, reference may be made to the prior art documents cited above and/or to other embodiments given in further detail below. Specifically, the test chemical may comprise one or more enzymes adapted to perform an enzymatic reaction in the presence of the analyte to be detected. Further, the test chemical may comprise one or more of: a colorants or dye; a mediator; a co-enzyme. Other embodiments are feasible. The test chemical may be adapted such that kinetics of the detection reaction may be tracked by monitoring the at least one optically detectable property, such as one or more of: a color, a remission, a reflection, a fluorescence, a phosphorescence. Thus, as an example, the test carrier may be adapted such that at least part of the test chemical is accessible for at least one optical measurement, such as through an opening of the substrate, through a window in the substrate and/or directly by inspecting the test field.

As used herein, the substrate generally may be an arbitrarily formed element which can be used as carrier for further elements. As an example, the form of the substrate may be selected from the group consisting of a strip, a tape, and a disc. Various embodiments are generally feasible.

The substrate may comprise a single layer setup or may comprise a multi-layer setup. Thus, the substrate may comprise one or more of a paper material, a plastic material, preferably a foil, a metal and a ceramic material. Further, combinations of materials are feasible. The substrate may comprise a multi-layer setup, such as by using a laminate. Further, the substrate may comprise one or more fluidic structures. For this purpose, two or more substrates may be provided, wherein a channel is disposed in between the substrate, such as by separating the substrate by one or more spacers. Additionally or alternatively, one or more fluidic structures on a surface of the substrate may be provided, such as by using one or more open capillary channels, such as one or more capillary slits. Various embodiments are feasible and, generally, are known in the art.

As outlined above, the test carrier may comprise at least one test field directly or indirectly applied to the substrate, such as applied to a surface of the substrate and/or integrated into the substrate, wherein the test field comprises the at least one test chemical. Therein, one single test field having at least one test chemical may be applied and/or a plurality of test fields having the same test chemistry and/or different types of test chemistry may be used.

The test carrier may comprise at least one application location, where a sample of the bodily fluid may be applied to. Consequently, the at least one application location may be a location in which a sample of the bodily fluid is applicable to the test carrier. In general the test carrier may comprise a plurality of application locations.

As outlined above, the at least one test chemical preferably forms at least one test field and/or is part of at least one test field. The test field may comprise a single-layer setup, comprising only one detection layer comprising the test chemical. Alternatively, the test field may have a multi-layer setup of at least two layers, wherein at least one detection layer comprising the at least one test chemical may be combined with one or more additional layers, such as one or more spreading layers and/or one or more separation layers and/or one or more pigment layers for providing an optical background, such as a white background, for improved optical measurements. Multi-layer setups of this type are known in the art. Thus, as an example, the test field may comprise at least one detection layer and, additionally, at least one separation layer (e.g. for separating blood cells) and/or optical layer comprising one or more pigments, such as one or more inorganic pigments, such as one or more metal oxides, preferably titanium dioxide.

For details of potential test chemicals, which may also be used within the present invention, reference may be made to J. Hoenes et al.: The Technology Behind Glucose Meters: Test Strips, Diabetes Technology & Therapeutics, Vol. 10, Supplement 1, 2008, S-10 to S-26. Further, reference may be made to WO 2010/094426 A1 and to WO 2010/094427 A1. Additionally or alternatively, the test substance as disclosed in WO 2007/012494 A1, WO 2009/103540 A1, WO 2011/012269 A2, WO 2011/012270 A1 or WO 2011/012271 A2 may be named, which is also referred to as the cNAD test substance. Further, reference may be made to EP 0 354 441 A2, EP 0 431 456 A1, EP 0 302 287 A2, to EP 0 547 710 A2 or to EP 1 593 434 A2. The test substances as disclosed in all these documents may also be used within the present invention. Other types of test elements and/or test substances are feasible and may be used within the present invention.

The light source, as used in method step b), general may be or may comprise one or more arbitrary light sources which are adapted to illuminate the test carrier. As used herein, "light" generally refers to electromagnetic waves in one or more of the visible, the ultraviolet and the infrared spectral range. Therein, the visible spectral range generally refers to a spectral range of 380 nm to 780 nm. The infrared spectral range generally refers to the spectral range of 780 nm to 1 mm, preferably 780 nm to 3.0 $\mu$m. The ultraviolet spectral range generally refers to the spectral range of 1 nm to 380 nm, preferably to the spectral range of 50 nm to 380 nm and more preferably the spectral range of 200 nm to 380 nm. Most preferably, the light source is adapted to emit light in the visible spectral range.

For example the light source may be a pulsed light source for example a light source selected from the group of: a light-emitting diode (LED); a laser, preferably a laser diode; an incandescent light source; a light bulb. In addition or alternatively several light sources, for example at least two light sources having differing emission wavelengths and/or having differing spectral properties, may be used.

As used herein, the term remitted light, as used in method step c), generally refers to light reflected by the test carrier, specifically by the test chemical and more specifically by at least one test field comprising the test chemical. The reflection may take place in a diffusive way. Generally, the reflection may fully or partially be elastic and/or inelastic. In some embodiments, method step c) is performed such that an angle of incidence of the illumination in method step b) differs from an angle of inspection in method step c), such that a direct reflection of light at least partially is excluded. A remission measurement as used in method steps b) and c) may be performed by illuminating the test carrier and/or a part thereof, and by detecting the reflected and/or scattered light from the test carrier. By performing this measurement, color changes in the test chemical on the test carrier, which may occur due to a progress of the detection reaction, may be detected. As a result of the measurement in method step c), a remission signal may be generated, such as a relative remission, as will be outlined in further detail below and as generally known in the art of optical detection.

The remitted light, or a part thereof, may be received by the at least one detector. The detector may be an arbitrary detector which is configured to receive light and to convert the light into one or more electric or electronic signals. The detector may comprise at least one light-sensitive element for detecting light propagating from the test carrier to the detector. The detector may generate one or more output detector signals, in particular at least one electronic signal, which may be further evaluated. The detector signal generally may be or may comprise an analog signal and/or a digital signal. Specifically, the detector signal may comprise an electric current signal and/or a voltage signal. The at least one detector signal may be a single detector signal or may comprise a plurality of detectors signals, such as by providing a continuous detector signal comprising continuously generated detector signals and/or detector signals generated at predetermined points in time and/or at a given detection frequency. The at least one detector signal may directly or indirectly be used in method step d). Thus, the detector signal may directly be processed in order to determine the concentration of the analyte. Additionally or alternatively, one or more preprocessing steps may be applied to the detector signal, in order to transform the detector signal as provided by the detector, also referred to as the raw detector signal or primary detector signal, into one or more secondary detector signals, such as by applying one or more of a filtering and/or an averaging process. In the following, when referring to the detector signal, both the option of using one or more primary detector signals and the option of using one or more secondary detector signals shall be implied.

In general, in certain embodiments the detector may comprise at least one light-sensitive element selected from the group consisting of: a photodiode; a photomultiplier; an imaging detector, in particular a camera chip such as a CMOS and/or a CCD chip. Other light-sensitive elements are feasible.

In method step d), the concentration of the analyte is determined by evaluating the at least one detector signal generated by the detector. As will be outlined in further detail below, the evaluation may be performed automatically, specifically by using at least one evaluation algorithm, in certain embodiments by using at least one data processing device adapted for automatically performing the evaluation algorithm, such as by using at least one software program.

The at least one light source is modulated by using at least two modulation frequencies. As used herein, a modulation of the light specifically may be or may imply a periodical change of at least one parameter of the light, such as at least one parameter selected from the group consisting of an amplitude, a frequency and a phase. As further used herein, the modulation frequency of a modulation is the frequency of the periodical change of the at least one parameter. Thus, mathematically speaking, the modulation may be a multiplication of the parameter to be modulated with a periodic function, such as one or more of the following functions:

$$a \cdot \exp[-i2\pi f\, t + \varphi],$$

$$a \cdot \sin[-2\pi f\, t + \varphi],$$

$$a \cdot \cos[-2\pi f\, t + \varphi],$$

wherein a denotes an amplitude of the modulation, wherein f denotes a frequency of the modulation and wherein φ denotes a phase of the modulation. Additionally or alternatively, the parameter to be modulated may be multiplied with a periodic delta function and/or may be multiplied with a periodic pulse function such as a rectangular function. Other types of modulation are feasible.

A modulation with at least two modulation frequencies, such as f1 and f2, generally refers to a doubling of the above-mentioned multiplication, i.e. a repeated modulation with two or more modulation frequencies.

The number of frequencies and/or of possible detection channels working in parallel may be limited by a processing power, such as an installed processing power, and/or by a required energy for mathematical calculation and processing. For a battery-driven analytical device, for instance, such as a battery-driven handheld meter, three frequencies per light source may be used. However, embodiments with more frequencies are feasible, such as for analytical devices connected to an external energy source.

As further used herein, a demodulation generally refers to the inverse process as compared to the modulation. Thus, as an example, a demodulation may imply multiplying or mixing the modulated function with a periodic function having a specific frequency, which also is referred to as the modulation frequency. Further, the demodulation may imply filtering or suppressing high-frequency components after performing the multiplication and/or mixing, in order to obtain low frequency components. For the first process, an electronic mixer or multiplier may be used, multiplying the signal to be demodulated with the at least one demodulation frequency, and for the latter process, a low-pass filter may be used. Thus, generally, the demodulation may imply a shifting or change of the signal from the original frequency of the light signal to a frequency which is evaluable and analyzable for the fault detection. The at least one light source may be modulated and/or demodulated by using two or more modulation frequencies.

The modulation frequencies may originate from a signal source, which may generate two or more control signals with two or more different frequencies, which may be used as modulation frequencies for modulation and/or a demodulation. As an example, the same signal source may be used for generating modulation frequencies both for the modulation and for demodulation. Generally, the modulation frequencies for modulation may be identical to the modulation frequencies of the demodulation. In general the signal source may be a signal generator, which e.g. generates a signal selected from the group of: a sinusoidal signal; a rectangular signal; a trapezoidal signal; a delta signal, preferably a periodic delta signal. In an optional mixer unit one control signal, for controlling the pulsed light source, may be generated by mixing these two control signals. The test carrier may be illuminated by this modulated light signal.

The control signals may have equal strength. As used herein, the term "strength" refers to an intensity level and/or amplitude level of a signal. The strength of the control signals may be equal to a strength of the detector signal. Thus, the probability of a faulty fault detection in case of low detector signals may be reduced.

The at least one light source comprises at least one first light source configured to be modulated by said at least one modulation device by at least two different modulation frequencies and at least one second light source configured to be modulated by said at least one modulation device by at least two different modulation frequencies being different from the at least two different modulation frequencies by which the first light source is modulated. Thus, it is possible to illuminate the track carrier by two light sources. This may be an advantage because an illumination of two different positions on the track carrier indicating a determination of two measurement values may be possible.

In general, when using more than one light source it may be possible to illuminate one, two or more different positions. Thus, at least one first light source may be adapted to illuminate at least one first position, and at least one second light source may be adapted to illuminate at least one second position, wherein the at least one first position and the at least one second position may fully or partially be identical or may fully or partially be non-identical, such as spatially separated and/or overlapping. For example, these different positions may be situated on the same test carrier and/or may be situated on different track carriers. Thus, it may be possible to illuminate two or more positions on two or more different test carriers and thus to determine two ore more measurement values of two or more different test carriers with the same device. The different test carriers may have different configurations such as a different geometry and/or different photometric properties.

The detector signal is demodulated with the at least two modulation frequencies in order to generate at least two demodulated detector signals, each demodulated detector signal corresponding to one of the modulation frequencies.

The process of demodulation can be understood as extracting the demodulated light signals from the detector signals. In the above-mentioned embodiment, wherein two light sources, each modulated by two modulation frequencies, may be used, the at least two demodulated detector signals may be generated for the modulation frequencies by which the first light source is modulated and at least two demodulated detector signals may be generated for the modulation frequencies by which the second light source is modulated.

The demodulation may comprise independently multiplying the detector signal with the modulation frequencies and filtering the results by using low-pass filters. A low-pass filter may be understood as an electronic component which is configured to pass signals with frequencies lower than a cutoff frequency, and to attenuate or suppress signals with higher frequencies.

The demodulation, before multiplying the detector signal with the modulation frequencies, may further comprise filtering the detector signal by using at least one band pass filter. A band pass filter is an electronic device configured to allow frequencies within a certain, predetermined range to pass and to rejects other frequencies outside this range. The band pass filter may be adjustable. Thus it may be possible to adjust the pass band to the used frequencies in the measurement.

The method comprises a fault detection based on a comparison of the at least two demodulated detector signals. The fault detection may be understood as recognizing disturbances, in particular disturbances due to one or more of ambient light, disturbances of one or more of the light sources and disturbances of one or more electronic components. Other disturbances are feasible. As used herein, a fault detection based on the comparison of the at least two demodulated detector signals generally refers to the fact that the fault detection takes into account the comparison by any suitable means, such as by implementing one result of the comparison into a fault detection algorithm as a variable and/or as a parameter. Thus, as an example, as will be outlined in further detail below, the fault detection may imply comparing one or more variables with at least one threshold, wherein the one or more variables may imply at least one result of the comparison.

Further the fault detection may provide a possibility to determine a reliable measurement value of a photometric measurement in presence of ambient light and/or rejecting the measurement. The fault detection may be an online fault detection which is performed permanently or repeatedly. The fault detection may be repeated once or several times during the photometric measurement.

The fault detection is based on a comparison of the at least two demodulated detector signals. Generally, as used herein, a comparison of the at least two demodulated detector signals refers to an algorithm adapted to generate a comparison result which depends on the magnitude of each of the demodulated detector signals and/or which depends on forming differences of two normed demodulated detector signals. Thus, as an example, the comparison may imply forming a difference between the at least two demodulated detector signals and/or may imply forming a quotient of the at least two demodulated detector signals. In case the demodulated detector signals each comprise a series of single values, the comparison may imply comparing a current or present value of the series. The comparison, as an example, may comprise at least one algorithm selected from the group consisting of: a comparison of at least one of the demodulated detector signals with at least another one of the demodulated detector signals; a comparison of at least one of the demodulated detector signals with at least one mean value of the demodulated detector signals; a comparison of at least one of the demodulated detector signals with at least one threshold value. Thus, generally, demodulated detector signals may be compared directly with each other or may be compared with at least one representative value representing e.g. a normal condition and/or representing the entity of the demodulated detector signals.

Generally, as outlined above, the fault detection may imply at least one threshold comparison. Thus, as an example, one or more of the demodulated detector signals and/or a difference between at least two demodulated detector signals and/or a quotient of two or more detector signals may directly or indirectly be compared to one or more thresholds.

The fault detection may comprise detecting faulty demodulated detector signals. A demodulated detector signal may be recognized as faulty, if the at least two generated demodulated detector signals show discrepancies of more than a predetermined tolerance. In an embodiment, more than two modulation frequencies may be used, for example three. Thus, one of the three demodulated detector signals may be recognized as faulty, if it differs from the other two demodulated detector signals by more than a predetermined tolerance, whereas the two other demodulated detector signals show similar values. In case, all demodulated detector signals differ by more than a predetermined tolerance, the whole set of demodulated signals may be detected as faulty.

The comparison of the at least two demodulated detector signals may comprise comparing at least a first one of the demodulated detector signals with at least a second one of the demodulated detector signals and determining that the first demodulated detector signal is faulty in case the first demodulated detector signal deviates from the second demodulated detector signal by more than a predetermined tolerance, preferably by a tolerance of 0-2%, more preferably by a tolerance of 0-1%.

Further the fault detection may comprise rejecting demodulated detector signals which are recognized as faulty demodulated detector signals, and may imply using only non-faulty demodulated detector signals for determining the concentration of the at least one analyte in the bodily fluid. If one of the demodulated detector signals is detected as faulty, this demodulated detector signal is rejected for determining the concentration of the at least one analyte in the bodily fluid. If the whole set of the demodulated detector signals are detected as faulty, the measurement is repeated with a new set of frequencies. In the latter case, the change of the set of frequencies may result in a certain settling time of the measurement devices, e.g. of the band pass filters. The demodulated detector signals each may be a sequence of measurement values, wherein rejecting the faulty demodulated detector signals may comprise a rejection algorithm selected from the group consisting of: rejecting a current measurement value which is determined to be faulty; rejecting the whole sequence of measurement values in case at least one of the measurement values is determined to be faulty. The method may be aborted in case all of the demodulated detector signals are determined to be faulty. Further each of the demodulated detector signals may comprise a sequence of single measurement values, wherein the fault detection may be based on a comparison of the single measurement values. A single measurement value can be understood as raw, non-evaluated and/or non-analyzed data. For example, single measurement values are issued by the detector every 20 ms, preferably every 10 ms.

Additionally or alternatively to the above mentioned fault detection may comprise detecting faulty demodulated detector signals. The fault detection may comprise determining a degree of faultiness for the demodulated detector signals which are determined to be faulty. Thus, it is possible that the at least one faulty demodulated detector signal may be used for determining the concentration of the analyte, wherein the degree of faultiness is taken into account.

The method may be performed repeatedly, wherein, in case in one of the repetitions of the method a faulty demodulated detector signal is found for a specific modulation frequency, said modulation frequency may be not used in a subsequent repetition of the method. In general it is possible to change, in case a faulty demodulated detector signal is found for a specific modulation frequency, to another frequency not used so far. However, then settling times will occur.

In the present invention, wherein two or more light sources, each modulated by at least two modulation frequencies, are used, the fault detection may be performed both for the demodulated detector signals for the modulation frequencies by which the first light source is modulated and for the demodulated detector signals for the modulation frequencies by which the second light source is modulated. Thus, for example, a reliable measurement value of the concentration of the analyte may be possible, even if in one set of demodulation frequencies for the modulation frequencies by which a light source, selected from the group of the first and the second light source, is modulated one or more demodulated detector signals are detected as faulty, and if in the other set of demodulation frequencies for the modulation frequencies by which the other light source, selected from the group of the first and the second light source, is modulated no faulty demodulated detector signal has been detected, by using only the light of the non-faulty light source.

The fault detection may be performed at least once before applying the sample of the bodily fluid to the test carrier. As outlined above, the method may comprise further steps, which specifically may fully or partially be performed before performing method steps a) - d), for example before an application of a sample of bodily fluid to a test carrier as proposed in method step a), and/or may be performed at least once independently from method step a).

Thus, the method may further comprise determining at least one dry empty value by evaluating the at least one detector signal generated by the detector before applying the sample of the bodily fluid to the test carrier. Performing a remission measurement of the test carrier before applying the sample of the bodily fluid, the so-called dry empty value, is well known to the skilled person. The fault detection may be performed at least once during determining the dry empty value. The dry empty value may be compared to reference values to determine a usability of the test carrier. In case the usability of the test carrier may be limited due to defects, e.g. aging defects caused by environmental influences such as humidity, light or temperature, it may be possible to reject the test carrier before applying a sample of bodily fluid to the test carrier and/or to adjust measurement values, e.g. one or more of the at least one detector signal and the determined concentration of the analyte.

The method further may comprise at least one position verification step, wherein the position verification step may comprise the following method steps:

   i) inserting the test carrier into the analytical device;
   ii) illuminating the test carrier by the at least one light source;
   iii) receiving light remitted by the test carrier by using the at least one detector;
   iv) determining at least one position of the test carrier within the analytical device by evaluating at least one detector signal generated by the detector, wherein the position comprises at least one of a location and/or an orientation of the test carrier.

The method steps may be performed in the given order, i.e. in the order i) - ii) - iii) - iv). However, other orders of the method steps are feasible, such as ii) - i) - iii) - iv). Thus, as an example, the test carrier may be or may comprise a strip-shaped test carrier or a test tape which may be inserted into a receptacle of the analytical device, before illuminating the test carrier by the at least one light source. Additionally or alternatively, the test carrier, such as a test tape and/or a test strip, may comprise one or more of at least one marking, at least one coating and/or at least one other item of information. The at least one item of information may contain at least one visually detectable item of information which may be detected by the at least one analytical device. The at least one item of information may contain at least one item of information regarding an appropriate use of the test carrier, such as at least one calibration information, and/or may contain at least one other item of information, such as a positioning mark or fiducial mark. The analytical device may be adapted for reading the at least one item of information, such as during insertion of the test carrier into the analytical device and/or within the analytical device. The analytical device may further be adapted for evaluating the at least one item of information and/or for controlling at least one process according to the at least one item of information. Thus, the analytical device may be adapted for controlling a positioning of the test carrier and/or for detecting whether the test carrier is correctly positioned. As an example, the analytical device may be adapted for illuminating a test tape and for detecting at least one marking on the test tape and/or for detecting at least one test field on the test tape, in order to control the positioning of the test tape and/or in order to detect whether the test tape is correctly positioned. A controlling of the positioning of the test tape may be performed by controlling an appropriate feeding mechanism of the analytical device, such as by controlling a motor for positioning the test tape. Thus, specifically in the latter case, an illumination of the test carrier may take place before or during insertion of the test carrier into the analytical device, such as for the purpose of monitoring the insertion process itself, such as a positioning process.

Further, one or more of the method steps may be performed in parallel and/or in a timely overlapping fashion, such as by performing method steps i) and ii) at least partially simultaneously and/or by performing method steps ii), iii) and iv) at least partially simultaneously. Further, one or more of the method steps may be performed repeatedly. Thus, as an example, method steps ii) and/or iii) may be performed repeatedly. Further, additional method steps may be present which are not listed.

The test carrier may be inserted into a receptacle of the analytical device. The test carrier and/or the analytical device and/or the light source and/or the detector specifically may be identical to the respective devices used in method steps a) - d). Additionally or alternatively, however, at least one additional light source and/or at least one additional detector is dedicated to the position verification step. For a description of possible embodiments and definitions of these devices, reference can be made to the above-mentioned devices used in method a) - d) and the above-mentioned analytical device according to the present invention. In general, other configurations of these devices may be possible.

The position verification step may be performed before performing method steps a) - d). The position verification step may comprise determining the location and/of the orientation of test carrier, including the possibility of determining a location or position of a part thereof such as a location or position of at least one test field of the test carrier, within the analytical device. As outlined above, the method steps i) - iv) may be performed at least once before applying the sample of bodily fluid to the test carrier, such as before performing the combination of method steps a) - d). In this embodiment the method steps i) - iv) may be performed at least once before applying the sample of bodily fluid to the test carrier in order to determine at least one position of the test carrier within the analytical device. The test carrier and/or the test field of the carrier may comprise a marking, e.g. a color marking and/or another arbitrary marking, e.g. with a known remission. As used herein, a "position" may be a location and/or orientation of the test carrier or a part thereof, such as of at least one test field of the test carrier, and/or the marking of the test carrier within the analytical device, e.g. within the receptacle of the analytical device. The remission of light of the test carrier, e.g. of a test strip, a test tape, a test disc, and an integrated test carrier, and/or the test field of the carrier may depend on its position within the analytical device. A proper alignment within the analytical device may be required for reliable measurement values.

After performing method steps i) - iv), the determined measurement values, e.g. one or more of the at least one detector signal and the determined concentration of the analyte, may be compared to reference values. In case the test carrier is aligned properly, the determined measurement values may correspond, within specified limits (such as within one or more thresholds), to the reference values.

The determination of the position may be performed once before applying the sample of the bodily fluid to the test carrier and/or during the photometric measurement. Hence, in case the test carrier is not aligned properly within the analytical device, it may be possible to abort the measurement at any desired time, e.g. before applying the sample to the test carrier, and/or to adjust measurement values, e.g. one or more of the at least one detector signal and the determined concentration of the analyte. In case it is determined that the test carrier is not aligned properly within the analytical device, an alignment may be performed by a user and/or automatically. Further in case at least one or more of the modulation frequencies may be susceptible, the susceptible modulation frequency may not be considered for the evaluation of the analyte concentration and/or a set of frequencies may be changed. Further, the fault detection may be performed at least once during the determination of the position of the test carrier.

In some embodiments, an ambient light fault detection step may be performed without using a test carrier. Herein, the method further may comprise at least one ambient light fault detection step, wherein the ambient light fault detection step may comprise the following method steps:

I. receiving ambient light by using the at least one detector;
II. evaluating at least one detector signal generated by the detector;
III. performing an ambient light fault detection by comparing the at least one detector signal generated by the detector with the modulation frequencies.

The method steps may be performed in the given order, i.e. in the order I. - II. - III.. However, other orders of the method steps are feasible, such as II. - I. - III.. Further, one or more of the method steps may be performed in parallel and/or in a timely overlapping fashion, such as by performing method steps I. and II. at least partially simultaneously. Further, one or more of the method steps may be performed repeatedly. Further, additional method steps may be present which are not listed.

In other embodiments, the ambient light detection may be performed after inserting the test carrier into the analytical device. Herein, the method further may comprise at least one ambient light fault detection step, wherein the ambient light fault detection step may comprise the following method steps:

I. inserting the test carrier into the analytical device;
II. illuminating the test carrier by the at least one light source;
III. receiving ambient light by using the at least one detector;
IV. evaluating at least one detector signal generated by the detector;
V. performing an ambient light fault detection by comparing the at least one detector signal generated by the detector with the modulation frequencies.

The method steps may be performed in the given order, i.e., in the order I. - II. - III. - IV. - V. However, other orders of the method steps are feasible, such as II. - I. - III. - IV. - V. Further, one or more of the method steps may be performed in parallel and/or in a timely overlapping fashion, such as by performing method steps I. and II. at least partially simultaneously and/or by performing method steps II., III. and IV. at least partially simultaneously. Further, one or more of the method steps may be performed repeatedly. Thus, as an example, method steps II. and/or III. may be performed repeatedly. Further, additional method steps may be present which are not listed.

In certain embodiments, a first ambient light fault detection step may be performed before inserting a test carrier into the analytical device and a second ambient light fault detection step may be performed after inserting a test carrier into the analytical device.

In all of these embodiments, the ambient light fault detection step may be based on a comparison of the at least one detector signal with the modulation frequencies. As used herein, when referring to the modulation frequencies in the context of the ambient light fault detection and the comparison of the at least one detector signal with the modulation frequencies, the modulation frequencies specifically may be or may comprise the frequency components of the detector signal at the respective modulation frequencies. Thus, a full or partial frequency analysis of the at least one detector signal may be performed, thereby deriving frequency components of the detector signal and, specifically, deriving frequency components of the detector signal at the modulation frequencies. Consequently, as used herein, the expression "comparing the at least one detector signal generated by the detector with the modulation frequencies" generally may refer to the fact that the above-mentioned frequency components may be evaluated, in order to determine whether at least one condition is fulfilled or not. Thus, as will be outlined in further detail below, the frequency components may be compared with one or more thresholds and/or with one or more tolerance ranges and/or with one or more conditions.

The ambient light fault detection step may be performed before performing method steps a) -d), for example before applying the sample of bodily fluid to the test carrier. Thus, generally, method steps I.-III. may be performed without inserting a test carrier into the analytical device, such as by leaving a receptacle of the analytical device empty. Specifically, the ambient light fault detection may be performed without a test carrier, such as without a test strip and/or without a test tape. Alternatively, a test carrier may be inserted into the analytical device, such as into at least one receptacle of the analytical device, and the ambient light fault detection step may comprise at least one step of inserting the test carrier into the analytical device. Thus, the ambient light fault detection optionally may take place in a realistic environment, with the test carrier inserted into the analytical device.

The ambient light fault detection may be performed without using a light source of the analytical device, such as by detecting the ambient light, only. Alternatively, the ambient light fault detection may be performed with additionally using at least one light source. Thus, in case no test carrier is inserted into the analytical device, the at least one light source may illuminate at least one empty receptacle of the analytical device and/or may illuminate at least one spot or region within the analytical device which normally is occupied by the test carrier and/or a part thereof, such as a test field of the test carrier. Thus, generally, the ambient light fault detection may further imply an illumination, such as an illumination of the analytical device and/or a part thereof by using at least one light source. Consequently, the at least one detector signal generated by the detector may contain at least one part due to the ambient light and at least one part due to light generated by at least one light source of the analytical device.

In case at least one test carrier is inserted into the analytical device for the purpose of the ambient light fault detection and/or during the ambient light fault detection, the process itself may further imply applying the sample of the bodily fluid to the test carrier. In the latter case, the ambient light fault detection specifically may be performed before applying the sample of bodily fluid to the test carrier, for example before detecting the at least one detector signal for the purpose of ambient light fault detection and/or before the light source is turned on and/or after the light source is turned on. Other options are feasible.

The performing of an ambient light fault detection by comparing the at least one detector signal generated by the detector with the modulation frequencies may further imply a comparison, such as a mathematical comparison, of the frequency components of the at least one detector signal at the modulation frequencies with one or more thresholds and/or conditions and/or tolerance ranges. For this purpose, the frequency components of the at least one detector signal may, each by itself or in a combined fashion, as raw values or after performing one or more preprocessing steps such as filtering or normalization, be compared with one or more thresholds and/or conditions and/or tolerance ranges. As an example, two or more of the frequency components of the at least one detector signal, as raw signals or after performing one or more preprocessing steps, be combined by using, e.g., a quotient and/or a difference between two or more of the frequency components, and the result of this mathematical operation may be compared with one or more thresholds and/or conditions and/or tolerance ranges. The ambient light fault detection may be dependent on the result of this comparison. Thus, as an example, in case one or more thresholds are exceeded and/or in case the result is found to be outside one or more tolerance ranges and/or in case one or more fault conditions are found to be fulfilled, a fault due to the ambient light may be detected, and, optionally, one or more appropriate actions may be taken, preferably automatically, such as providing a warning and/or preventing further measurement. Additionally or alternatively, in case the at least one detector signal, one or more of a plurality of detector signals or at least one signal component of the at least one detector signal are found to be faulty, such as due to disturbances by ambient light, the respective faulty measurement signal or measurement signal component or the respective modulation frequency may be excluded from the method for determining the concentration of the at least one analyte in the bodily fluid. Thus, as an example, the ambient light fault detection may determine if one or more of the at least two modulation frequencies are such that demodulated detector signals for the respective at least one modulation frequencies are faulty and may exclude the respective at least one modulation frequency, which may be denoted as a "faulty modulation frequency" from the determination of the analyte concentration. Thus, as an example, the faulty modulation frequency may be replaced by another modulation frequency for the determination of the analyte concentration. Additionally or alternatively, the at least one demodulated detector signal for the faulty modulation frequency, which may also be referred to as a "faulty demodulated detector signal", may be excluded from further evaluation and/or may be used with a lower weighting factor as compared to other demodulated detector signals. The demodulated detector signals may be used for determining an average value of the analyte concentration, such as a weighted average value, specifically a sliding average or a weighted sliding average. The averaging may take place before, during or after determining the analyte concentration. Thus, the determining of the analyte concentration may be performed on the basis of one, more than one or all of the demodulated detector signals, such as by using a common correlation between the demodulated detector signals as input variables and the analyte concentration as output variables and/or by independently determining the analyte concentration by independently using demodulated detector signals as input variables and, subsequently, combining the independent results such as by determining a mean or average value or a weighted average value. Therein, in case one or more demodulated detector signals are determined as faulty demodulated detector signals during the ambient light fault detection, the one or more faulty demodulated detector signals may be excluded from the determination of the analyte concentration and/or may be used at a lower weight, such as by using lower weighting factors in the weighted average as compared to non-faulty demodulated detector signals.

The test carrier may be inserted into the receptacle of the analytical device. The test carrier, and/or the analytical device, and/or the light source, and/or the detector may be identical to the devices used in method steps a) - d). Additionally or alternatively, however, at least one additional light source and/or at least one additional detector is dedicated to the ambient light fault detection step. For a description of possible embodiments and definitions of these devices, reference can be made to the above-mentioned devices used in method a) - d) and the above-mentioned analytical device according to the present invention. In general, other configurations of these devices may be possible.

The expression "ambient light" may be understood as light emitted by arbitrary light sources which are present during performing the proposed method, e.g. sun light, light of artificial light sources. The ambient light fault detection step may be performed, such as before performing method steps a) - d), in order to determine the contribution of one or more possible modulation frequencies within the ambient light.

The detector may receive the ambient light and may generate at least one detector signal. The at least one detector signal generated by the detector may be evaluated with respect to the contribution of one or more possible modulation frequencies within the ambient light. The evaluation may comprise comparing the at least one detector signal to the at least one modulation frequency and/or a set of modulation frequencies, which may be used for modulating the light source. In case the ambient light shows contributions of at least one modulation frequencies, which may be used for modulating the light source, the at least one modulation frequency may not be considered for the evaluation of the analyte concentration and/or the set of frequencies may be changed.

As outlined above, method step d) implies determining the concentration of the analyte by evaluating the at least one detector signal generated by the detector. As used herein, an evaluation of at least one detector signal generally refers to an arbitrary algorithm for deriving a concentration of the analyte from the at least one detector signal. The algorithm may be or may comprise an analytical algorithm such as an evaluation function.

Additionally or alternatively, any other type of algorithm may be used, such as a lookup table or any other algorithm which is adapted to assign a specific value of the detector signal a concentration of the analyte. These algorithms generally are known to the skilled person. As an example, an end value of a measurement curve comprising a sequence of detector signals may be used as a characteristic value, and the analyte concentration may be derived thereof. Thus, as an example, the algorithm as disclosed in EP 0 821 234 and in US 2002/0146835 A1 may be used, in which the measurement curve directly or indirectly is compared with one or more thresholds. Thus, as an example, EP 0 821 234 B1 discloses a method in which a slope of the measurement curve is determined by deriving difference values of colors and comparing these difference values with a predetermined threshold. Thereby, an end point of the detection reaction may be determined. Similarly, in US 2002/0146835 A1, an end point is determined by calculating an intermediate analyte level of the testing element at predetermined intervals and calculating a ratio value corresponding to the $(n)^{th}$ measurement to an $(n-5)^{th}$ measurement. When two consecutive ratio values are less than or equal to a predetermined value, the end point is deemed to be reached, and the final analyte level can be determined.

Further, several evaluation algorithms using one or more fitting algorithms are known in the art, in which the measurement curve comprising the detector signals is analyzed by using one or more fit functions. Thus, in WO 2011/061257 A1, a method and a device for analyzing a body fluid are disclosed, in which a photometric measurement curve is measured. A transmission behavior of an optical transmission system is controlled by detecting measured values at two different measurement wavelengths. Further, fit functions are generated for the two measurement curves, and, by extrapolating fit curves, an offset of the measurement values is determined. In US 2008/0087819 A1, a method for analyzing a fluid sample is disclosed, in which, again, two different wavelengths are used for deriving two measurement curves. The measurement curves are fitted by using an exponential rise with a subsequent exponential fall, by performing an appropriate fit algorithm having two different types of temporal constants.

In WO 01/25760 A1, a timing-independent method for determining a proper time for measurement of a reaction between a sample fluid and a reagent on an analyte strip is disclosed. Therein, a measurement curve of a characteristic of a matrix, to which sample fluid is applied, is periodically measured both before and after application of the sample fluid. Subsequently, a transformation is made of this measurement curve into a function which is independent in time or at most various linearly in time. The second derivative of the transformed function is then analyzed to determine when the second derivative falls below a predetermined threshold. At this point in time, the transformed function will yield the analyte concentration in the sample fluid. In EP 1 413 883 A1, a method of reducing analysis time of end point-type reaction profiles is disclosed. For this purpose, a detection reaction is initiated, obtaining at least three measurements, at three different points in time, of a value or level of an observable associated with the detection reaction. Subsequently, an end point value for the observable is estimated from the measurements, by using an appropriate fit function. In WO 2006/138226 A2, an arrangement and an algorithm for calculating the concentration of an analyte contained in a sample are disclosed. Therein, a color change rate of a test chemical is detected, and a hematocrit is derived from the color change rate. An appropriate correction factor indicative of the hematocrit is used for correcting a glucose concentration.

These algorithms and/or any other evaluation algorithm known to the skilled person may be used for performing method step d), wherein, in certain embodiments, only non-faulty detector signals are used in method step d) in order to determine the analyte concentration. Step d) of the method further may be performed by using a data processing device and/or computer. For example the fault detection may be performed by using a data processing device and/or computer, in particular the comparison of the demodulated detector signals.

In addition, it may be possible to store information of fault detection for certain frequencies and/or reoccurring fault detections for certain frequencies. Thus, the method may imply storing information on a previous fault detection in at least one data memory, for use in future measurements. As an example, information on one or more modulation frequencies which are known to be faulty and/or which are known to be non-faulty may be stored in at least one data memory. Thus, it may be possible to start the measurement with non-susceptible or non-faulty frequencies. The method may be performed such that, automatically or by manual adjustment by a user, one or more modulation frequencies are chosen which are known to be non-faulty, such as from previous measurements. Thus, an analytical device performing the method may be adapted to offer two or more modulation frequencies to a user and/or may be adapted to automatically choose, such as without the need of a user input, two or more reliable modulation frequencies, which are known to be non-faulty, at least from previous measurements.

In certain embodiments, the method further may comprise one or more or even all of the following method steps, which, in an embodiment, may be performed before performing method step a), i.e. before applying the sample of the bodily fluid to the test carrier:

    i. inserting the test carrier into an analytical device;
    ii. initiating the fault detection;
    iii. acquiring the dry empty value.

As outlined above with regard to method steps a) through d), these method steps i.-iii. may be performed in the given order and/or in any other feasible order, as will be evident to the skilled person. Further, one or more or even all of these additional method steps may be combined with one or more of method steps a) through d).

For further details of the analytical device, reference may be made to the disclosure of the second aspect of the present invention as given below.

The method according to the invention allows for performing the fault detection basically at any reasonable time of the photometric measurement, as will be evident to the skilled person. Further it may be possible to avoid faulty or inaccurate measurement values caused by disturbances of ambient light by changing from the used, susceptible frequencies to frequencies, which are not susceptible. This is achieved on the one hand by comparing the demodulated detector signals at a very early stage of the measurement e.g. during the determination of the dry empty value, instead of comparing the evaluated value of the concentration of the analyte. Further this is achieved by using more than one set of modulation frequencies. Thus, it is possible to change the set frequencies in case of fault detection. In general the amount of frequency changes is not limited. However, the settling time of the used measurement devices may have to be considered.

In addition, the described fault detection at the very early stage of the measurement provides the possibility to secure the robustness of the determined concentration value of the analyte by detecting disturbances before applying the sample of bodily fluid to the test carrier. A pre-selection of the set of frequencies may be possible by performing one or more frequency changes and/or one or more fault detections before applying the sample of bodily fluid to the test carrier. Thus, the set of frequencies, which has the lowest susceptibility, can be preselected.

For example, the method may be performed with the two modulation frequencies 1,488 kHz and 1,587 kHz. If the demodulated detector signal generated during the determination of the dry empty value for this set of frequencies shows discrepancies above a certain threshold value, this pair may be detected as faulty and may be rejected. In this case, the set of frequencies may be changed to another set of frequencies, e.g. 1,302 kHz and 1,389 kHz. If again this set of frequencies shows discrepancies above a certain threshold, this pair may be detected as faulty and may be rejected too. Again the second set of frequencies may be changed to another set of frequencies, e.g. 1,645 kHz and 1,754 kHz. In case the third set of frequencies is not detected faulty, the sample is applied to the carrier and measurement of the concentration of the analyte will start.

Additionally or alternatively to the comparison of the demodulated detector signals with one threshold value, two or more threshold values may be established. The at least one threshold value may be or may comprise at least one predetermined threshold value and/or may be or may comprise at least one adjustable threshold value, which may be adjustable manually and/or automatically. For example, one narrow threshold value, e.g. a deviation of the demodulated detector signals of 0.5 %, and one wider threshold value, e.g. a deviation of the demodulated detector signals of 1-2%. In case the deviation of the demodulated detector signals lies within the narrow threshold range, a warning may be generated to display to a user that the measurement is questionable. Instead, if the deviation of the demodulated detector signals lies within the wider threshold range, a change of the susceptible frequency or an abortion of the measurement may be performed.

In another embodiment, more than two frequencies may be used for the modulation of the light source. For example, three, four or more than four frequencies may be used for the modulation of the light source, e.g. $f_i$, $f_{ii}$, $f_{iii}$.. In this embodiment the three demodulated detector signals for the three modulation frequencies may be generated and may be compared. Hence, if only one of the frequencies is susceptible, it may be possible to use only non-faulty demodulated detector signals for the evaluation of the analyte concentration. A resulting detector signal may be determined as mean value of the non-faulty demodulated detector signals. For example, if frequency $f_i$ is susceptible and $f_{ii}$ and $f_{iii}$ are not, it may be possible to consider only the frequencies $f_{ii}$ and $f_{iii}$ for evaluation of the analyte concentration. As an example, the method may be aborted only if all of the three demodulated detector signals show different values. Thus, it may be possible to determine the concentration of the analyte even in case of fault detection for one of the frequencies without changing the whole set of frequencies.

For example, in one embodiment two light-emitting diodes may be used as light sources. The signal of one of the light sources may be modulated with three frequencies, e.g. $f_{1a}$ = 977 Hz, $f_{1b}$ = 1465 Hz and $f_{1c}$ = 1953 Hz. The signal of the other light source may be modulated with three other frequencies, e.g. $f_{2a}$ = 1172 Hz, $f_{2b}$ = 1563 Hz and $f_{2c}$ = 2344 Hz. During the fault detection, in a first step the demodulated detector signals of $f_{1a}$ and $f_{1b}$ may be compared. In a second step, these demodulated detector signals may be compared with the demodulated detector signal of $f_{1c}$. The mean detector output signal may be evaluated only from equal values, wherein equal indicates equal within a certain threshold. For the evaluation of the mean detector output signal at least two values may be needed. If all demodulated detector signals differ more than the predefined threshold value, an error value may be generated and a change to other than the susceptible frequencies may be performed. An equal method may be applied to frequencies of the second light source. The two determined mean values may be further evaluated to determine the concentration of the analyte. At this late stage of the measurement it may be further possible to compare the two determined concentrations of the analyte. If the two measurement results are not equal a warning and/or an error value may be issued.

In a further aspect of the present invention, an analytical device for determining a concentration of at least one analyte in a bodily fluid comprising the features of appended claim 16 is disclosed. As used herein, generally, an analytical device refers to a device which is adapted to perform at least one analysis in order to determine the concentration of one or more analytes in the body fluid. The analytical device may be a hand-held device or may be a stationary or portable device.

At least one sample of the bodily fluid is applicable to the test carrier. In order to achieve this aspect, the analytical device may be adjusted such that the sample of the body fluid may be applied to the test carrier before inserting the test carrier into the receptacle and/or in a state in which the test carrier is inserted into the receptacle. In the first case, the receptacle may be designed such that the test carrier with the sample applied thereto may be inserted into the receptacle. In the latter case, the receptacle may be designed such that at least one portion of the test carrier having at least one application position is accessible to a user, in order to allow for the application of the sample.

The analytical device may be adapted to perform the method according to the method described in the first aspect of the invention. For a description of possible embodiments and definitions, reference can be made to the above-mentioned method according to the present invention.

As used here, the receptacle may be an arbitrarily formed device, which is configured to allow for an insertion of the test carrier. The receptacle may further be adapted to enable the application of the sample of bodily fluid to the test carrier. The receptacle generally may comprise at least one means for holding the test carrier in at least one predetermined position. Thus, as an example, the receptacle may comprise one or more of a slot, a guiding structure, a holder, a chamber. Other types of receptacles are feasible. The receptacle may be adapted to hold the test carrier in position during the photometric measurement. The receptacle may comprise at least one opening adapted to insert the test carrier into the receptacle, such as one or more of a slotted opening, a rectangular opening, a round opening.

The analytical device further comprises at least one light source adapted for illuminating the test carrier and at least one detector adapted for receiving light remitted by the test carrier. For potential embodiments of the light source, reference may be made to the definitions and embodiments given above or given in further detail below.

The analytical device further comprises at least one evaluation unit adapted for determining the concentration of the analyte by evaluating at least one detector signal generated by the detector. As used herein, an evaluation unit generally refers to a device or system of multiple devices which is or are configured to evaluate the at least one detector signal generated by the detector. For example the evaluation unit may comprise a data processing device and/or computer. Thus, as an example, a microprocessor may be integrated in the evaluation unit. Additionally or alternatively, external data-processing devices may be included into the analytical device, such as one or more personal computers, one or more computer networks or one or more other types of data processing devices.

The analytical device further comprises at least one modulation device adapted for modulating the light source by using at least two different modulation frequencies. As used herein, a modulation device generally refers to at least one device which is configured to perform a modulation as defined above. Thus, the modulation device generally may be adapted to periodically modulated at least one parameter of the at least one light source and/or of the light emitted by the at least one light source.

The signal source may be adapted to generate one or more control signals having at least two modulation frequencies. In particular, the modulation device is adapted to modulate the light source by using at least three modulation frequencies.

The analytical device further comprises at least one demodulation device adapted for the demodulating the detector signal with the at least two different modulation frequencies in order to generate at least two demodulated detector signals, each demodulated detector signal corresponding to one of the modulation frequencies. As used herein, the demodulation device generally refers to a device which is configured to perform a demodulation process as defined above. Thus, the demodulation device may be adapted to demodulate a signal which was modulated by at least two different modulation frequencies. The demodulation device may be adapted such that the demodulation comprises independently multiplying the detector signal with the one or more modulation frequencies and filtering the results by using one or more low-pass filters. In addition, the demodulation device may be adapted such that the demodulation, before multiplying the detector signal with the modulation frequencies, comprises filtering the detector signal by using at least one band pass filter. In certain embodiments, the band pass filter is adjustable, manually and/or automatically.

The demodulation device may comprise at least one lock-in amplifier. For example, the lock-in amplifier may be or may comprise a single phase lock-in amplifier. The single phase lock-in amplifier may comprise a single lock-in-structure using one reference signal. In an embodiment, the lock-in amplifier may be a digital dual phase lock-in amplifier, such as in order to be phase independent. The digital dual phase lock-in amplifier may comprise a dual lock-in-structure. The dual lock-in structure may comprise two single lock-in-structures each comprising a reference signal. A reference signal may have and/or may be modulated with the same modulation frequency by which the light source is modulated. One of the reference signals of the dual lock-in structure may be shifted, for example, the reference signal may be shifted by 90°. An output signal of the dual phase lock-in amplifier may depend on the square root of the sum of the squared individual signals. As used herein, the term "lock-in amplifier" may be used as a synonym for dual phase lock-in amplifier.

The analytical device further comprises at least one fault detection device adapted for performing a fault detection based on a comparison of the at least two demodulated detector signals. The fault detection device is a device or system of devices configured to perform the above described fault detection. The fault detection device may comprise a data processing device and/or computer. The fault detection device may fully or partially be part of the evaluation device and/or may fully or partially be embodied as a separate device. Further, the fault detection device may be adapted to perform the fault detection as an online fault detection, which may be performed permanently or repeatedly. As used herein, online fault detection generally refers to a fault detection which is performed during a measurement procedure of the photometric measurement, such as during determining the analyte concentration.

For details of potential embodiments of the fault detection, reference may be made to the disclosure of the method as given above and/or as given in further detail below. The fault detection device may be adapted such that the comparison of the at least two demodulated detector signals comprises at least one algorithm selected from the group consisting of: a comparison of at least one of the demodulated detector signals with at least another one of the demodulated detector signals; a comparison of at least one of the demodulated detector signals with at least one mean value of the demodulated detector signals; a comparison of at least one of the demodulated detector signals with at least one threshold value. For example, the fault detection device may be adapted such that the comparison of the at least two demodulated detector signals comprises comparing at least a first one of the demodulated detector signals with at least a second one of the demodulated detector signals and determining that the first demodulated detector signal is faulty in case the first demodulated detector signal deviates from the second demodulated detector signal by more than a predetermined tolerance, preferably by a tolerance of 0-2%, more preferably by a tolerance of 0-1%.

In general, the fault detection device may be adapted such that the fault detection comprises detecting faulty demodulated detector signals. In certain embodiments, the fault detection device may be adapted such that the fault detection further comprises rejecting the faulty demodulated detector signals and using only nonfaulty demodulated detector signals for determining the concentration of the at least one analyte in the bodily fluid. As used within the present invention, a rejection generally refers to a process of preventing a further use of a demodulated detector signal which is recognized to be faulty. The rejection may be an automatic rejection automatically preventing the use of the faulty demodulated detector signal. Additionally or alternatively, the rejection may be a semi-automatic and/or manual rejection, such as by providing a warning to a user indicating that a specific modulation frequency or at demodulated detector signal is faulty.

In particular, the demodulation device may be adapted such that the demodulated detector signals each comprise a sequence of measurement values, wherein rejecting the faulty demodulated detector signals may comprise a rejection algorithm selected from the group consisting of: rejecting a current measurement value which is determined to be faulty; rejecting the whole sequence of measurement values in case at least one of the measurement values is determined to be faulty. The analytical device may be adapted such that determining the concentration of the analyte is aborted in case all of the demodulated detector signals are determined to be faulty. In addition, the analytical device may be adapted such, that in case the determination of the concentration of an analyte is aborted, an output indicating the abortion is issued.

Further additional or alternatively, the fault detection device may be adapted such that the fault detection comprises determining a degree of faultiness for the demodulated detector signals which are determined to be faulty. The evaluation unit may be adapted such that at least one faulty demodulated detector signal is used for determining the concentration of the analyte, wherein the degree of faultiness is taken into account.

The at least one light source comprises at least one first light source configured to be modulated by said at least one modulation device by at least two different modulation frequencies and at least one second light source configured to be modulated by said at least one modulation device by at least two different modulation frequencies being different from the at least two different modulation frequencies by which the first light source is modulated. For each light source at least two signals each with a modulation frequency may be generated by the signal source. In the mixer unit, one control signal for controlling may be generated by mixing, in particular adding up, the two signals for each light source. Each of the two light sources may be controlled by one of the generated control signals and may illuminate the test carrier. The remitted light may be detected by a detector. In this embodiment, the demodulation device may be adapted such that at least two demodulated detector signals are generated for the modulation frequencies by which the first light source is modulated and wherein at least two demodulated detector signals are generated for the modulation frequencies by which the second light source is modulated. Hence, the fault detection device may be adapted such that the fault detection is performed both for the demodulated detector signals for the modulation frequencies by which the first light source is modulated and for the demodulated detector signals for the modulation frequencies by which the second light source is modulated.

In another embodiment, the demodulation device may be adapted such that each of the demodulated detector signals comprises a sequence of single measurement values, wherein the fault detection device may be adapted such that the fault detection is based on a comparison of the single measurement values. The advantage of comparing single measurement data is, that the comparison takes place at an early stage of the measurement and that these measurement data may be available fast as no evaluation steps like calculations and/or integrations are determined.

The fault detection device may be adapted such that the fault detection is performed at least once before applying the sample of the bodily fluid to the test carrier. The analytical device may be adapted to determine at least one dry empty value by evaluating the at least one detector signal generated by the detector before applying the sample of the bodily fluid to the test carrier. The fault detection device may be adapted such that fault detection is performed at least once during determining the dry empty value. Thus, the fault detection may be performed before the determination of the concentration of the analyte in the bodily fluid. Hence, it may possible to abort the measurement before applying the sample to the test carrier, so that the inserted test carrier is still usable and not rejected.

In a further aspect of the invention, an analytical system for determining at least one analyte in a bodily fluid comprising the features of appended claim 17 is disclosed. Thus, generally, an analytical system, as used herein, refers to a combination of at least one analytical device and at least one test carrier as independent entities which either may be handled independently or which may be handled in combination and which may cooperate in order to determine the concentration of the at least one analyte in the at least one body fluid. For a description of possible embodiments and definitions, reference can be made to the above-mentioned method and the above-mentioned analytical device according to the present invention.

The analytical system comprises at least one test carrier. The test carrier may be selected from the group consisting of a test strip, a test tape, a test disc, and an integrated test carrier having at least one test chemical and at least one lancet element.

As used here, the lancet element may be an arbitrary element which is configured to puncture and/or to cut into the skin of a user to generate at least one sample of the bodily fluid. The lancet element may comprise one or more of a round tip, a sharp tip, a flat tip, a needle and an edge. The lancet element may comprise further elements, for example elements which are configured to sample and/or transport the sample of bodily fluid, in particular a capillary.

The test carrier may comprise at least one substrate and at least one test chemical applied to the substrate, wherein the test chemical may be adapted to perform at least one detection reaction in the presence of the analyte to be detected and to change at least one optically detectable property due to the detection. The optically detectable property may be an arbitrary optical property which changes due to the detection reaction and, the measurement of which may therefor provide at least one item of information regarding a progress, an extent or a status of the detection reaction. In certain embodiments, the at least one optically detectable information is selected from the group consisting of a color; a reflection property such as a remission and a fluorescence of the test chemical. Other embodiments are feasible.

As described in detail above, the light source uses at least two frequencies. The described devices and/or system allow reliable measurement results even in case of disturbances. The described devices and/or system are operational even in case of using and/or detecting faulty frequencies.

An example further discloses and proposes a computer program including computer-executable instructions for performing the method according to the present invention and/or parts thereof, in one or more of the embodiments enclosed herein, when the program is executed on a processor residing within the analytical device, on a computer or computer network. Specifically, the computer program may be stored on a computer-readable data carrier, e.g. on a ROM such as a Flash-ROM, such as a computer-readable data carrier and/or a ROM (such as a Flash-ROM) of the test carrier. Thus, specifically, one, more than one or even all of method steps a) to d) as indicated above may be performed by using a processor residing within the analytical device, a computer or a computer network, preferably by using a computer program. Specifically, one or more of determining the concentration of the analyte as disclosed in method step d), the demodulation of the at least one detector signal and the fault detection may be performed by using a processor residing within the analytical device, a computer or a computer network.

An example further discloses and proposes a computer program product having program code means, in order to perform the method according to the present invention and/or parts thereof, in one or more of the embodiments enclosed herein when the program is executed on a processor residing within the analytical device, on a computer or computer network. Specifically, the program code means may be stored on a computer-readable data carrier.

Further, an example discloses and proposes a data carrier having a data structure stored thereon, which, after loading into a data storage residing within the analytical device, a computer or computer network, such as into a working memory or main memory of the computer or computer network, may execute the method and/or parts thereof, according to one or more of the embodiments disclosed herein.

An example further proposes and discloses a computer program product with program code means stored on a machine-readable carrier, in order to perform the method and/or parts thereof, according to one or more of the embodiments disclosed herein, when the program is executed on a processor residing within the analytical device, on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, an example proposes and discloses a modulated data signal which contains instructions readable by a processor residing within the analytical device, a computer system or computer network, for performing the method and/or parts thereof, according to one or more of the embodiments disclosed herein.

Preferably, referring to the computer-implemented examples, one or more of the method steps or even all of the method steps of the method according to one or more of the embodiments disclosed herein may be performed by using a processor residing within the analytical device, a computer or computer network. Thus, generally, any of the method steps including provision and/or manipulation of data may be performed by using a processor residing within the analytical device, a computer or computer network. Generally, these method steps may include any of the method steps, typically except for method steps requiring manual work, such as providing the samples and/or certain aspects of performing the actual measurements.

Specifically, examples further disclose:

- An analytical device, a computer or computer network comprising at least one processor, wherein the processor is adapted to perform the method and/or parts thereof, according to one of the embodiments described in this description,
- a computer loadable data structure that is adapted to perform the method and/or parts thereof, according to one of the embodiments described in this description while the data structure is being executed on a computer,
- a computer program, wherein the computer program is adapted to perform the method and/or parts thereof, according to one of the embodiments described in this description while the program is being executed on a processor residing within the analytical device or on a computer,
- a computer program comprising program means for performing the method and/or parts thereof, according to one of the embodiments described in this description while the computer program is being executed on a processor residing within the analytical device, on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method and/or parts thereof, according to one of the embodiments described in this description after having been loaded into a main and/or working storage of an analytical device, a computer or of a computer network, and
- a computer program product having program code means, wherein the program code means can be stored or are stored on a storage medium, for performing the method and/or parts thereof, according to one of the embodiments described in this description, if the program code means are executed on an analytical device, on a computer or on a computer network.

Summarizing the findings of the present invention, the following embodiments are particularly referred to:

Embodiment 1: A method for determining a concentration of at least one analyte in a bodily fluid, the method comprising:

a) applying a sample of the bodily fluid to a test carrier;
b) illuminating the test carrier by using at least one light source;
c) receiving light remitted by the test carrier by using at least one detector;
d) determining the concentration of the analyte by evaluating at least one detector signal generated by the detector;

wherein the at least one light source is modulated by using at least two different modulation frequencies, wherein the detector signal is demodulated with the at least two different modulation frequencies in order to generate at least two demodulated detector signals, each demodulated detector signal corresponding to one of the modulation frequencies, wherein the method comprises a fault detection based on a comparison of the at least two demodulated detector signals, wherein the at least one light source comprises at least one first light source being modulated by at least two different modulation frequencies and at least one second light source being modulated by at least two different modulation frequencies being different from the at least two different modulation frequencies by which the first light source is modulated.

Embodiment 2: The method according to the preceding embodiment, wherein the fault detection is an online fault detection which is performed permanently or repeatedly.

Embodiment 3: The method according to any of the preceding embodiments, wherein the at least one light source is modulated by using at least three different modulation frequencies.

Embodiment 4: The method according to any of the preceding embodiments, wherein the comparison of the at least two demodulated detector signals comprises at least one algorithm selected from the group consisting of: a comparison of at least one of the demodulated detector signals with at least another one of the demodulated detector signals; a comparison of at least one of the demodulated detector signals with at least one mean value of the demodulated detector signals; a comparison of at least one of the demodulated detector signals with at least one threshold value.

Embodiment 5: The method according to any of the preceding embodiments, wherein the comparison of the at least two demodulated detector signals comprises comparing at least a first one of the demodulated detector signals with at least a second one of the demodulated detector signals and determining that the first demodulated detector signal is faulty in case the first demodulated detector signal deviates from the second demodulated detector signal by more than a predetermined tolerance, preferably by a tolerance of 0-2%, more preferably by a tolerance of 0-1%.

Embodiment 6: The method according to any of the preceding embodiments, wherein the fault detection comprises detecting faulty demodulated detector signals.

Embodiment 7: The method according to the preceding embodiment, wherein the fault detection further comprises rejecting the faulty demodulated detector signals and using only non-faulty demodulated detector signals for determining the concentration of the at least one analyte in the bodily fluid.

Embodiment 8: The method according to the preceding embodiment, wherein the demodulated detector signals each are a sequence of measurement values, wherein rejecting the faulty demodulated detector signals comprises a rejection algorithm selected from the group consisting of: rejecting a current measurement value which is determined to be faulty; rejecting the whole sequence of measurement values in case at least one of the measurement values is determined to be faulty.

Embodiment 9: The method according to any of the three preceding embodiments, wherein the method is aborted in case all of the demodulated detector signals are determined to be faulty.

Embodiment 10: The method according to any of the four preceding embodiments, wherein the fault detection comprises determining a degree of faultiness for the demodulated detector signals which are determined to be faulty.

Embodiment 11: The method according to the preceding embodiment, wherein at least one faulty demodulated detector signal is used for determining the concentration of the analyte, wherein the degree of faultiness is taken into account.

Embodiment 12: The method according to any of the six preceding embodiments, wherein the method is performed repeatedly, wherein, in case in one of the repetitions of the method a faulty demodulated detector signal is found for a specific modulation frequency, said modulation frequency is not used in a subsequent repetition of the method.

Embodiment 13: The method according to any one of the preceding embodiment, wherein at least two demodulated detector signals are generated for the modulation frequencies by which the first light source is modulated and wherein at least two demodulated detector signals are generated for the modulation frequencies by which the second light source is modulated.

Embodiment 14: The method according to the preceding embodiment, wherein the fault detection is performed both for the demodulated detector signals for the modulation frequencies by which the first light source is modulated and for the demodulated detector signals for the modulation frequencies by which the second light source is modulated.

Embodiment 15: The method according to any of the preceding embodiments, wherein each of the demodulated detector signals comprises a sequence of single measurement values, wherein the fault detection is based on a comparison of the single measurement values.

Embodiment 16: The method according to any of the preceding embodiments, wherein the fault detection is performed at least once before applying the sample of the bodily fluid to the test carrier.

Embodiment 17: The method according to the preceding embodiment, wherein the method further comprises determining at least one dry empty value by evaluating the at least one detector signal generated by the detector before applying the sample of the bodily fluid to the test carrier.

Embodiment 18: The method according to the preceding embodiment, wherein the fault detection is performed at least once during determining the dry empty value.

Embodiment 19: The method according to any one of the preceding embodiments, wherein the method further comprises at least one position verification step, wherein the position verification step comprises the following method steps:

> i) inserting the test carrier into an analytical device;
> ii) illuminating the test carrier by the at least one light source;
> iii) receiving light remitted by the test carrier by using the at least one detector;
> iv) determining at least one position of the test carrier within the analytical device by evaluating at least one detector signal generated by the detector,

wherein the position comprises at least one of a location and/or an orientation of the test carrier.

Embodiment 20: The method according to any one of the preceding embodiments, wherein the method further comprises at least one ambient light fault detection step, wherein the ambient light fault detection step comprises the following method steps:

> I. receiving light remitted from the test carrier by using the at least one detector;
> II. evaluating at least one detector signal generated by the detector;
> III. performing an ambient light fault detection by comparing the at least one detector signal generated by the detector with the modulation frequencies.

Embodiment 21: The method according to any one of the preceding embodiments, wherein the method further comprises at least one ambient light fault detection step, wherein the ambient light fault detection step comprises the following method steps:

> I. inserting the test carrier into the analytical device;
> II. illuminating the test carrier by the at least one light source;
> III. receiving ambient light by using the at least one detector;
> IV. evaluating at least one detector signal generated by the detector;
> V. performing an ambient light fault detection by comparing the at least one detector signal generated by the detector with the modulation frequencies.

Embodiment 22: The method according to any of the preceding embodiments, wherein the demodulation comprises independently multiplying the detector signal with the modulation frequencies and filtering the results by using low pass filters.

Embodiment 23: The method according to the preceding embodiment, wherein the demodulation, before multiplying the detector signal with the modulation frequencies, comprises filtering the detector signal by using at least one band pass filter.

Embodiment 24: The method according to the preceding embodiment, wherein the band pass filter is adjustable.

Embodiment 25: The method according to any of the preceding embodiments, wherein the test carrier is selected from the group consisting of a test strip, a test tape, a test disc, and integrated test carrier having at least one test chemical and at least one lancet element. Embodiment 26: The method according to any of the preceding embodiments, wherein the test carrier comprises at least one substrate and at least one test chemical applied to the substrate, wherein the test chemical is adapted to perform at least one detection reaction in the presence of the analyte to be detected and to change at least one optically detectable property due to the detection reaction.

Embodiment 27: The method according to any of the preceding embodiments, wherein step d) is performed by using a data processing device and/or computer.

Embodiment 28: The method according to any of the preceding embodiments, wherein the fault detection is performed by using a data processing device and/or computer.

Embodiment 29: The method according to one of the preceding embodiments, wherein the method further comprises at least one of the following method steps, such as before performing method step a):

> i. inserting the test carrier into an analytical device;
> ii. initiating the fault detection;
> iii. acquiring a dry empty value.

Embodiment 30: An analytical device for determining a concentration of at least one analyte in a bodily fluid, the analytical device comprising at least one receptacle for receiving at least one test carrier, wherein at least one sample of the bodily fluid is applicable to the test carrier, the analytical device further comprising at least one light source adapted for illuminating the test carrier, the analytical device further comprising at least one detector adapted for receiving light remitted by the test carrier, the analytical device further comprising at least one evaluation unit adapted for determining the concentration of the analyte by evaluating at least one detector signal generated by the detector, the analytical device further comprising at least one modulation device adapted for modulating the light source by using at least two different modulation frequencies, the analytical device further comprising at least one demodulation device adapted for the demodulating the detector signal with the at least two modulation frequencies in order to generate at least two demodulated detector signals, each demodulated detector signal corresponding to one of the modulation frequencies, the analytical device further comprising at least one fault detection device adapted for performing a fault detection based on a comparison of the at least two demodulated detector signals, wherein the at least one light source comprises at least one first light source configured to be modulated by said at least one modulation device by at least two different modulation frequencies and at least one second light source configured to be modulated by said at least one modulation device by at least two different modulation frequencies being different from the at least two different modulation frequencies by which the first light source is modulated.

Embodiment 31: The analytical device according to the preceding embodiment, wherein the analytical device is adapted to perform the method according to any of the preceding method embodiments.

Embodiment 32: The analytical device according to any of the preceding embodiments referring to a device, wherein the evaluation unit comprises a data processing device and/or computer.

Embodiment 33: The analytical device according to any of the preceding embodiments referring to a device, wherein the fault detection device comprises a data processing device and/or computer.

Embodiment 34: The analytical device according to any of the preceding embodiments referring to a device, wherein the modulation device comprises at least one signal source.

Embodiment 35: The analytical device according to the preceding embodiment, wherein the signal source is adapted to generate control signals having the at least two modulation frequencies.

Embodiment 36: The analytical device according to any of the preceding embodiments referring to a device, wherein the fault detection device is adapted to perform the fault detection as an online fault detection which is performed permanently or repeatedly.

Embodiment 37: The analytical device according to any of the preceding embodiments referring to a device, wherein the modulation device is adapted to modulate the light source by using at least three different modulation frequencies.

Embodiment 38: The analytical device according to any of the preceding embodiments referring to a device, wherein the fault detection device is adapted such that the comparison of the at least two demodulated detector signals comprises at least one algorithm selected from the group consisting of: a comparison of at least one of the demodulated detector signals with at least another one of the demodulated detector signals; a comparison of at least one of the demodulated detector signals with at least one mean value of the demodulated detector signals; a comparison of at least one of the demodulated detector signals with at least one threshold value.

Embodiment 39: The analytical device according to any of the preceding embodiments referring to a device, wherein the fault detection device is adapted such that the comparison of the at least two demodulated detector signals comprises comparing at least a first one of the demodulated detector signals with at least a second one of the demodulated detector signals and determining that the first demodulated detector signal is faulty in case the first demodulated detector signal deviates from the second demodulated detector signal by more than a predetermined tolerance, preferably by a tolerance of 0-2%, more preferably by a tolerance of 0-1%.

Embodiment 40: The analytical device according to any of the preceding embodiments referring to a device, wherein the fault detection device is adapted such that the fault detection comprises detecting faulty demodulated detector signals.

Embodiment 41: The analytical device according to the preceding embodiment, wherein the fault detection device is adapted such that the fault detection further comprises rejecting the faulty demodulated detector signals and using only non-faulty demodulated detector signals for determining the concentration of the at least one analyte in the bodily fluid.

Embodiment 42: The analytical device according to the preceding embodiment, wherein the demodulation device is adapted such that the demodulated detector signals each are a sequence of measurement values, wherein rejecting the faulty demodulated detector signals comprises a rejection algorithm selected from the group consisting of: rejecting a current measurement value which is determined to be faulty; rejecting the whole sequence of measurement values in case at least one of the measurement values is determined to be faulty.

Embodiment 43: The analytical device according to any of the three preceding embodiments, wherein the analytical device is adapted such that determining the concentration of the analyte is aborted in case all of the demodulated detector signals are determined to be faulty.

Embodiment 44: The analytical device according to any of the four preceding embodiments, wherein the fault detection device is adapted such that the fault detection comprises determining a degree of faultiness for the demodulated detector signals which are determined to be faulty.

Embodiment 45: The analytical device according to the preceding embodiment, wherein the evaluation unit is adapted such that at least one faulty demodulated detector signal is used for determining the concentration of the analyte, wherein the degree of faultiness is taken into account.

Embodiment 46: The analytical device according to any of the preceding embodiments referring to a device, wherein the at least one light source comprises at least one first light source configured to be modulated by said at least one modulation device by at least two different modulation frequencies and at least one second light source configured to be modulated by said at least one modulation device by at least two different modulation frequencies being different from the at least two different modulation frequencies by which the first light source is modulated.

Embodiment 47: The analytical device according to the preceding embodiment, wherein the demodulation device is adapted such that at least two demodulated detector signals are generated for the modulation frequencies by which the first light source is modulated and wherein at least two demodulated detector signals are generated for the modulation frequencies by which the second light source is modulated.

Embodiment 48: The analytical device according to the preceding embodiment, wherein the fault detection device is adapted such that the fault detection is performed both for the demodulated detector signals for the modulation frequencies by which the first light source is modulated and for the demodulated detector signals for the modulation frequencies by which the second light source is modulated.

Embodiment 49: The analytical device according to any of the preceding embodiment referring to a device, wherein the demodulation device is adapted such that each of the demodulated detector signals comprises a sequence of single measurement values, wherein the fault detection device is adapted such that the fault detection is based on a comparison of the single measurement values.

Embodiment 50: The analytical device according to any of the preceding embodiment referring to a device, wherein the fault detection device is adapted such that the fault detection is performed at least once before applying the sample of the bodily fluid to the test carrier.

Embodiment 51: The analytical device according to the preceding embodiment, wherein the analytical device is adapted to determine at least one dry empty value by evaluating the at least one detector signal generated by the detector before applying the sample of the bodily fluid to the test carrier.

Embodiment 52: The analytical device according to the preceding embodiment, wherein the fault detection device is adapted such that fault detection is performed at least once during determining the dry empty value.

Embodiment 53: The analytical device according to any of the preceding embodiment referring to a device, wherein the demodulation device is adapted such that the demodulation comprises independently multiplying the detector signal with the modulation frequencies and filtering the results by using low pass filters.

Embodiment 54: The analytical device according to the preceding embodiment, wherein the demodulation device is adapted such that the demodulation, before multiplying the detector signal with the modulation frequencies, comprises filtering the detector signal by using at least one band pass filter.

Embodiment 55: The analytical device according to the preceding embodiment, wherein the band pass filter is adjustable.

Embodiment 56: An analytical system for determining a concentration of at least one analyte in a bodily fluid, the analytical system comprising the analytical device according to any of the preceding device embodiments, the analytical system further comprising at least one test carrier.

Embodiment 57: The analytical system according to the preceding embodiment, wherein the test carrier is selected from the group consisting of a test strip, a test tape, a test disc, and an integrated test carrier having at least one test chemical and at least one lancet element.

Embodiment 58: The analytical system according to any of the two preceding embodiment, wherein the test carrier comprises at least one substrate and at least one test chemical applied to the substrate, wherein the test chemical is adapted to perform at least one detection reaction in the presence of the analyte to be detected and to change at least one optically detectable property due to the detection reaction.

SHORT DESCRIPTION OF THE FIGURES

Further optional features and embodiments of the invention will be disclosed in more detail in the subsequent description of the particular embodiments of the invention, preferably in conjunction with the dependent claims. Therein, the respective optional features may be realized in an isolated fashion as well as in any arbitrary feasible combination, as the skilled person will realize. The scope of the invention is not restricted by the particular embodiments. The embodiments are schematically depicted in the Figures. Therein, identical reference numbers in the Figures refer to identical or functionally comparable elements.

In the Figures:

Figure 1     shows a schematic view of an exemplary embodiment of a proposed analytical system comprising an exemplary embodiment of a proposed analytical device and a test carrier; and

Figure 2     shows an exemplary embodiment of a signal of a detector.

DETAILED DESCRIPTION OF THE EMBODIMENTS

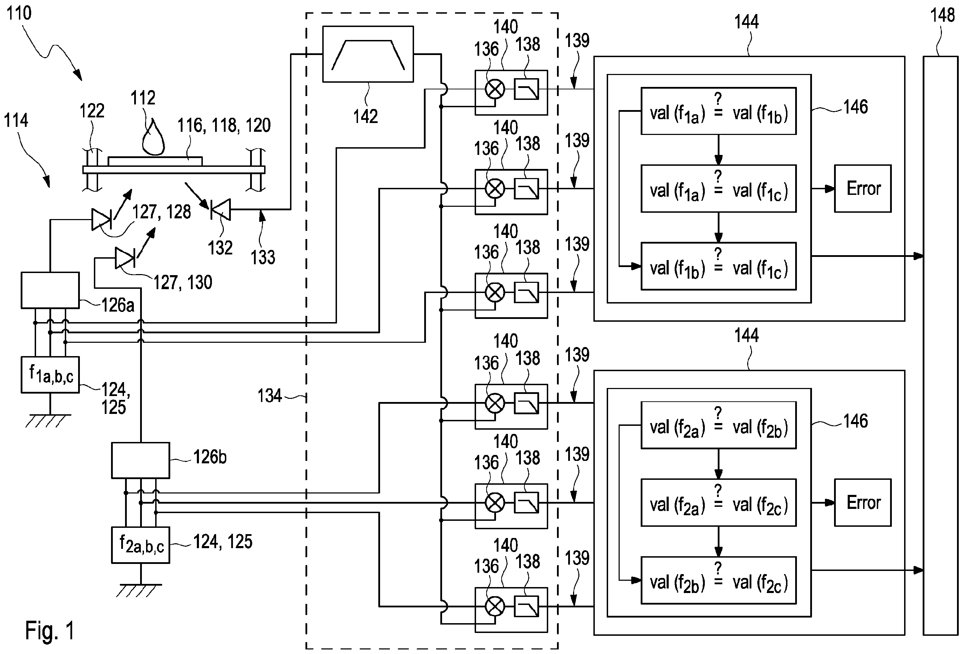

In Figure 1, a schematic view of an analytical system 110 for determining a concentration of at least one analyte in a bodily fluid 112 is depicted. The analytical system 110 comprises an analytical device 114. Further the analytical system 110 comprises a test carrier 116, which, in this exemplary embodiment, may be embodied as a test strip. The test carrier 116 may comprise at least one substrate 118 and at least one test chemical 120, which can be applied to and/or integrated into the substrate 118. The test chemical 120 is adapted to change at least one optically detectable property due to a detection reaction. The analytical device 114 comprises a receptacle 122 in which the test carrier 116 may be inserted.

The analytical device 114 may comprise at least one, preferably two or more, modulation devices 124. Each of the modulation devices 124 may comprise at least one signal source 125. Each of the signal sources 125 may generate a different set of control signals, such as three or more control signals, having different modulation frequencies, such as having three or more different modulation frequencies.

In the embodiment depicted in Figure 1, the three modulation frequencies of the three control signals of the first modulation device 124 are denoted by $f_{1a}$, $f_{1b}$ and $f_{1c}$, whereas the three modulation frequencies of the three control signals of the second modulation device 124 are denoted by $f_{2a}$, $f_{2b}$ and $f_{2c}$.

For further reference, devices and processes concerning the first set of frequencies may be referred to as a first channel, whereas devices, frequencies and processes concerning the second set of frequencies may be referred to as a second channel. The analytical device may further comprise two or more mixer units 126. For potential details of the mixer units 126, reference may be made to EP 1 912 058 B1. Control signals generated by the first channel may be transferred into a mixer unit 126a, and the control signal of the second channel may be transferred into another mixer unit 126b. In both of the channels, a control signal may be generated by mixing the three control signals in the mixer unit 126.

The analytical device 114 comprises at least one light source 127. The light source 127 comprises as depicted in figure 1 a first light source 128 and a second light source 130. The first light source 128 may be controlled by the control signal of the first channel, whereas the second light source 130 may be controlled by the control signal of the second channel. The test carrier 116 is illuminated by the light originating from the first light source 128 and the second light source 130. The test carrier 116 remits the light which is detected by a detector 132. The detector 132 may convert the light signals into an electronic signal which, in the following, is also referred to as a detector signal and is symbolically referred to by reference number 133 in Figure 1. The detector signal 133 is modulated by, in this embodiment, six modulation frequencies.

The analytical device 114 further comprises at least one demodulation device 134. The demodulation device 134 is adapted for demodulating the signal of the detector 132. The demodulation device 134 as shown in Figure 1 may comprise three multiplication devices 136 and three low pass filters 138 for each of the two channels. In each of the multiplication devices 136 the detector signal may be multiplied or mixed with one of the modulation frequencies, wherein each of the modulation frequencies is only used once. In each of the low-pass filters 138 the result of the previous multiplication may be filtered. Thus, at output ports of the respective low-pass filters 138, demodulated detector signals may be provided which, symbolically, in Figure 1 are denoted by reference numbers 139. In an alternative nomenclature, each of the outputs providing the demodulated detector signals 139 may form a channel of the demodulation device 134.

As depicted in Figure 1, one of the multiplication devices 136 in combination with one of the low-pass filters 138 can be realized as lock-in amplifier 140. The demodulation device 134 may further comprise a band pass filter 142 which is configured to filter the detector signal before passing the detector signal to the lock-in amplifiers 140.

The analytical device 114 further comprises, e.g. for each of the two channels, a fault detection device 144, which is configured to perform a fault detection. The fault detection device 144 may be adapted for performing a comparison procedure which, in Figure 1, is symbolically denoted by reference number 146. During the comparison procedure 146, the demodulated detector signals 139, indicated as val ($f_{1a, b, c}$) and val ($f_{2a, b, c}$), may be compared. For example in the first channel, in case one of the demodulated detector signal of val ($f_{1a}$), val ($f_{1b}$) and val ($f_{1c}$) differs more than a certain threshold from the other demodulated detector signals, the demodulated detector signal of the respective susceptible frequency may be rejected from further evaluation. As long as two demodulated detector signals are equal at least within a predetermined or adjustable tolerance, an average value may be calculated from these demodulated detector signals for further evaluation. If all demodulated detector signals differ from each other by more than a predetermined or adjustable threshold, an error value may be issued and/or a photometric measurement may be restarted with a new set of frequencies. The comparison process 146 may be performed with the data processing device and/or a computer.

The fault detection may be performed as an online fault detection. Thus, the fault detection may be performed repeatedly or permanently during the photometric measurement. Further the fault detection may be performed before applying a sample of bodily fluid 112 to the test carrier 116, e.g. during a determination of a dry empty value.

In addition, the analytical device 114 of Figure 1 comprises an evaluation unit 148 which is adapted for determining the concentration of the analyte by evaluating an input of the fault detection device 144 of each of the two channels. In the evaluation unit 148 the concentration of the analyte determined in the two channels may be compared and an error value may be issued if the determined values differ from each other more than a certain threshold. For details of the evaluation unit 148, reference may be made to the disclosure given above and/or to the prior art documents as cited above.

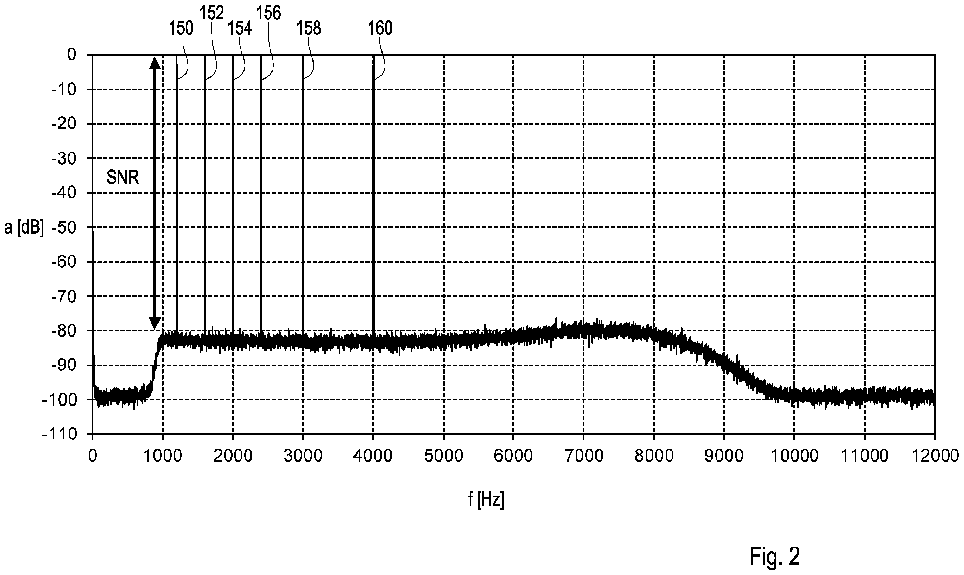

In Figure 2 an exemplary embodiment of a signal 133 of the detector 132 is depicted. A dependency of the frequency f[Hz] over the attenuation a [dB] is depicted. The signal 133 may comprise six modulation frequencies 150 - 160. The signal 133 may be determined by the detector 132 before demodulation by the demodulation device 136 and before determining a measurement result by the evaluation unit 148. The signal 133 may comprise no disturbances. The used six modulation frequencies 150 - 160 may have equal strength. In addition, a signal to noise ratio SNR of the signal 133 is shown. The SNR may be large enough to differentiate each of the modulation frequencies 150 - 160 from noise of the detector 132.

List of reference numbers

**[0138]**

| | |
|---|---|
| 110 | analytical system |
| 112 | bodily fluid |
| 114 | analytical device |
| 116 | test carrier |
| 118 | substrate |
| 120 | test chemical |
| 122 | receptacle |
| 124 | modulation devices |
| 125 | signal source |
| 126 | mixer unit |
| 127 | light source |
| 128 | first light source |
| 130 | second light source |
| 132 | detector |
| 133 | detector signal |
| 134 | demodulation device |
| 136 | multiplication device |
| 138 | low-pass filter |
| 139 | demodulated detector signals |
| 140 | lock-in amplifier |
| 142 | band pass filter |
| 144 | fault detection device |
| 146 | comparison procedure |
| 148 | evaluation unit |
| 150 | modulation frequency |
| 152 | modulation frequency |
| 154 | modulation frequency |
| 156 | modulation frequency |
| 158 | modulation frequency |
| 160 | modulation frequency |

**Claims**

1. A method for determining a concentration of at least one analyte in a bodily fluid (112), the method comprising:

   a) applying a sample of the bodily fluid (112) to a test carrier (116);
   b) illuminating the test carrier (116) by using at least one light source (127);
   c) receiving light remitted by the test carrier (116) by using at least one detector (132);
   d) determining the concentration of the analyte by evaluating at least one detector signal (133) generated by the detector (132);

   wherein the at least one light source (127) is modulated by using at least two different modulation frequencies, wherein the detector signal (133) is demodulated with the at least two different modulation frequencies in order to generate at least two demodulated detector signals, each demodulated detector signal corresponding to one of the modulation frequencies,

wherein the method comprises a fault detection based on a comparison of the at least two demodulated detector signals, **characterized in that** the at least one light source (127) comprises at least one first light source (128) being modulated by at least two different modulation frequencies and at least one second light source (130) being modulated by at least two different modulation frequencies being different from the at least two different modulation frequencies by which the first light source (128) is modulated.

2. The method according to the preceding claim, wherein the fault detection is an online fault detection which is performed permanently or repeatedly.

3. The method according to any one of the preceding claims, wherein the at least one light source (127) is modulated by using at least three different modulation frequencies.

4. The method according to any one of the preceding claims, wherein the fault detection comprises detecting faulty demodulated detector signals.

5. The method according to the preceding claim, wherein the fault detection further comprises rejecting the faulty demodulated detector signals and using only non-faulty demodulated detector signals for determining the concentration of the at least one analyte in the bodily fluid (112).

6. The method according to the preceding claim, wherein at least one faulty demodulated detector signal is used for determining the concentration of the analyte, wherein the degree of faultiness is taken into account.

7. The method according to any one of the preceding claims, wherein at least two demodulated detector signals are generated for the modulation frequencies by which the first light source (128) is modulated and wherein at least two demodulated detector signals are generated for the modulation frequencies by which the second light source (130) is modulated.

8. The method according to the preceding claim, wherein the fault detection is performed both for the demodulated detector signals for the modulation frequencies by which the first light source (128) is modulated and for the demodulated detector signals for the modulation frequencies by which the second light source (130) is modulated.

9. The method according to any one of the preceding claims, wherein the fault detection is performed at least once before applying the sample of the bodily fluid (112) to the test carrier (116).

10. The method according to the preceding claim, wherein the method further comprises determining at least one dry empty value by evaluating the at least one detector signal generated by the detector (132) before applying the sample of the bodily fluid (112) to the test carrier (116).

11. The method according to any one of the preceding claims, wherein the method further comprises at least one position verification step, wherein the position verification step comprises the following method steps:

i) inserting the test carrier (116) into an analytical device (114);
ii) illuminating the test carrier (116) by the at least one light source (127);
iii) receiving light remitted by the test carrier (116) by using the at least one detector (132);
iv) determining at least one position of the test carrier (116) within the analytical device (114) by evaluating at least one detector signal (133) generated by the detector (132), wherein the position comprises at least one of a location and/or an orientation of the test carrier (116).

12. The method according to any one of the preceding claims, wherein the method further comprises at least one ambient light fault detection step, wherein the ambient light fault detection step comprises the following method steps:

I. receiving ambient light by using the at least one detector (132);
II. evaluating at least one detector signal (133) generated by the detector (132);
III. performing an ambient light fault detection by comparing the at least one detector signal (133) generated by the detector (132) with the modulation frequencies.

13. The method according to any one of the preceding claims, wherein the demodulation comprises independently multiplying the detector signal (133) with the modulation frequencies and filtering the results by using low pass filters (138).

14. The method according to the preceding claim, wherein the demodulation, before multiplying the detector signal (133) with the modulation frequencies, comprises filtering the detector signal (133) by using at least one band pass filter (142).

**15.** The method according to any one of the preceding claims, wherein the method further comprises at least one of the following method steps:

- inserting the test carrier (116) into an analytical device (114);
- initiating the fault detection;
- acquiring a dry empty value

**16.** An analytical device (114) for determining a concentration of at least one analyte in a bodily fluid (112), the analytical device (114) comprising at least one receptacle (122) for receiving at least one test carrier (116), wherein at least one sample of the bodily fluid (112) is applicable to the test carrier (116), the analytical device (114) further comprising at least one light source (127) adapted for illuminating the test carrier (116), the analytical device (114) further comprising at least one detector (132) adapted for receiving light remitted by the test carrier (116), the analytical device (114) further comprising at least one evaluation unit (148) adapted for determining the concentration of the analyte by evaluating at least one detector signal (133) generated by the detector (132), the analytical device (114) further comprising at least one modulation device (124) adapted for modulating the light source (127) by using at least two different modulation frequencies, the analytical device (114) further comprising at least one demodulation device (134) adapted for the demodulating the detector signal (133) with the at least two different modulation frequencies in order to generate at least two demodulated detector signals, each demodulated detector signal corresponding to one of the modulation frequencies, the analytical device (114) further comprising at least one fault detection device (144) adapted for performing a fault detection based on a comparison of the at least two demodulated detector signals, **characterized in that** the at least one light source (127) comprises at least one first light source (128) configured to be modulated by said at least one modulation device (124) by at least two different modulation frequencies and at least one second light source (130) configured to be modulated by said at least one modulation device (124) by at least two different modulation frequencies being different from the at least two different modulation frequencies by which the first light source (128) is modulated.

**17.** An analytical system (110) for determining a concentration of at least one analyte in a bodily fluid (112), the analytical system (110) comprising the analytical device (114) according to the preceding claim, the analytical system (110) further comprising at least one test carrier (116).

**Patentansprüche**

**1.** Verfahren zur Bestimmung einer Konzentration von mindestens einem Analyt in einer Körperflüssigkeit (112), wobei das Verfahren umfasst:

a) Aufbringen einer Probe der Körperflüssigkeit (112) auf einen Testträger (116);
b) Beleuchten des Testträgers (116) unter Verwendung von mindestens einer Lichtquelle (127);
c) Empfangen des von dem Testträger (116) zurückgesendeten Lichts unter Verwendung von mindestens einem Detektor (132);
d) Bestimmen der Konzentration des Analyts durch Evaluieren von mindestens einem Detektorsignal (133), das von dem Detektor (132) erzeugt wurde;

wobei die mindestens eine Lichtquelle (127) unter Verwendung von mindestens zwei verschiedenen Modulationsfrequenzen moduliert wird, wobei das Detektorsignal (133) mit den mindestens zwei verschiedenen Modulationsfrequenzen demoduliert wird, um mindestens zwei demodulierte Detektorsignale zu erzeugen, wobei jedes demodulierte Detektorsignal einer der Modulationsfrequenzen entspricht, wobei das Verfahren eine Fehlererfassung basierend auf einem Vergleich der mindestens zwei demodulierten Detektorsignale umfasst, **dadurch gekennzeichnet, dass** die mindestens eine Lichtquelle (127) mindestens eine erste Lichtquelle (128) umfasst, die mit mindestens zwei verschiedenen Modulationsfrequenzen moduliert wird, und mindestens eine zweite Lichtquelle (130), die mit mindestens zwei verschiedenen Modulationsfrequenzen moduliert wird, die sich von den mindestens zwei verschiedenen Modulationsfrequenzen unterscheiden, mit denen die erste Lichtquelle (128) moduliert wird.

**2.** Verfahren nach dem vorhergehenden Anspruch, wobei die Fehlererfassung eine Online-Fehlererfassung ist, die ständig oder wiederholt durchgeführt wird.

**3.** Verfahren nach einem der vorhergehenden Ansprüche, wobei die mindestens eine Lichtquelle (127) unter Verwendung von mindestens drei verschiedenen Modulationsfrequenzen moduliert wird.

**4.** Verfahren nach einem der vorhergehenden Ansprüche, wobei die Fehlererfassung das Erfassen fehlerhafter demodulierter Detektorsignale umfasst.

**5.** Verfahren nach dem vorhergehenden Anspruch, wobei die Fehlererfassung ferner das Ablehnen fehlerhafter demodulierter Detektorsignale und das Verwenden von nur nicht fehlerhaften demodulierten Detektorsignalen zum Bestimmen der Konzentration des mindestens einen Analyts in der Körperflüssigkeit (112) umfasst.

6. Verfahren nach dem vorhergehenden Anspruch, wobei das mindestens eine fehlerhafte demodulierte Detektorsignal zur Bestimmung der Konzentration des Analyts verwendet wird, wobei der Grad der Fehlerhaftigkeit berücksichtigt wird.

7. Verfahren nach einem der vorhergehenden Ansprüche, wobei mindestens zwei demodulierte Detektorsignale für die Modulationsfrequenzen erzeugt werden, mit denen die erste Lichtquelle (128) moduliert wird, und wobei mindestens zwei demodulierte Detektorsignale für die Modulationsfrequenzen erzeugt werden, mit denen die zweite Lichtquelle (130) moduliert wird.

8. Verfahren nach dem vorhergehenden Anspruch, wobei die Fehlererfassung sowohl für die demodulierten Detektorsignale für die Modulationsfrequenzen durchgeführt wird, mit denen die erste Lichtquelle (128) moduliert wird, als auch für die demodulierten Detektorsignale für die Modulationsfrequenzen, mit denen die zweite Lichtquelle (130) moduliert wird.

9. Verfahren nach einem der vorhergehenden Ansprüche, wobei die Fehlererfassung mindestens einmal vor dem Aufbringen der Probe der Körperflüssigkeit (112) auf den Testträger (116) durchgeführt wird.

10. Verfahren nach dem vorhergehenden Anspruch, wobei das Verfahren ferner das Bestimmen von mindestens einem Trockenleerwert durch Evaluieren des mindestens einen Detektorsignals, das von dem Detektor (132) erzeugt wurde, vor dem Aufbringen der Probe der Körperflüssigkeit (112) auf den Testträger (116) umfasst.

11. Verfahren nach einem der vorhergehenden Ansprüche, wobei das Verfahren ferner mindestens einen Positionsverifizierungsschritt umfasst, wobei der Positionsverifizierungsschritt die folgenden Verfahrensschritte umfasst:

    i) Einsetzen des Testträgers (116) in ein Analysegerät (114);
    ii) Beleuchten des Testträgers (116) mit der mindestens einen Lichtquelle (127);
    iii) Empfangen des von dem Testträger (116) zurückgesendeten Lichts unter Verwendung des mindestens einen Detektors (132);
    iv) Bestimmen von mindestens einer Position des Testträgers (116) innerhalb des Analysegeräts (114) durch Evaluieren von mindestens einem Detektorsignal (133), das von dem Detektor (132) erzeugt wurde, wobei die Position mindestens eine von einer Position und/oder einer Ausrichtung des Testträgers (116) umfasst.

12. Verfahren nach einem der vorhergehenden Ansprüche, wobei das Verfahren ferner mindestens einen Umgebungslichtfehlererfassungsschritt umfasst, wobei der Umgebungslichtfehlererfassungsschritt die folgenden Verfahrensschritte umfasst:

    I. Empfangen von Umgebungslicht unter Verwendung des mindestens einen Detektors (132);
    II. Evaluieren von mindestens einem Detektorsignal (133), das von dem Detektor (132) erzeugt wurde;
    III. Durchführen einer Umgebungslichtfehlererfassung durch Vergleichen des mindestens einen Detektorsignals (133), das von dem Detektor (132) erzeugt wurde, mit den Modulationsfrequenzen.

13. Verfahren nach einem der vorhergehenden Ansprüche, wobei die Demodulation das unabhängige Multiplizieren des Detektorsignals (133) mit den Modulationsfrequenzen und das Filtern der Ergebnisse unter Verwendung von Tiefpassfiltern (138) umfasst.

14. Verfahren nach dem vorhergehenden Anspruch, wobei die Demodulation vor dem Multiplizieren des Detektorsignals (133) mit den Modulationsfrequenzen das Filtern des Detektorsignals (133) unter Verwendung eines Tiefpassfilters (142) umfasst.

15. Verfahren nach einem der vorhergehenden Ansprüche, wobei das Verfahren ferner mindestens einen der folgenden Verfahrensschritte umfasst:

    - Einsetzen des Testträgers (116) in ein Analysegerät (114);
    - Initiieren der Fehlererfassung;
    - Erfassen eines Trockenleerwerts.

16. Analysegerät (114) zur Bestimmung einer Konzentration von mindestens einem Analyt in einer Körperflüssigkeit (112), wobei das Analysegerät (114) mindestens ein Fach (122) zum Aufnehmen von mindestens einem Testträger (116) umfasst, wobei mindestens eine Probe der Körperflüssigkeit (112) auf den Testträger (116) aufgebracht werden kann, wobei das Analysegerät (114) ferner mindestens eine Lichtquelle (127) umfasst, die zum Beleuchten des Testträgers (116) eingerichtet ist, wobei das Analysegerät (114) ferner mindestens einen Detektor (132) umfasst, der zum Empfangen von Licht, das von dem Testträger (116) zurückgesendet wird, eingerichtet ist, wobei das Analysegerät (114) ferner mindestens eine Evaluierungseinheit (148) umfasst, die zum Bestimmen der Konzentration des Analyts durch Evaluieren von mindestens einem Detektorsignal (133), das von dem Detektor (132) erzeugt wurde, eingerichtet ist,

wobei das Analysegerät (114) ferner mindestens ein Modulationsgerät (124) umfasst, das zum Modulieren der Lichtquelle (127) unter Verwendung von mindestens zwei verschiedenen Modulationsfrequenzen eingerichtet ist, wobei das Analysegerät (114) ferner mindestens ein Demodulationsgerät (134) umfasst, das zum Demodulieren des Detektorsignals (133) mit den mindestens zwei verschiedenen Modulationsfrequenzen eingerichtet ist, um mindestens zwei demodulierte Detektorsignale zu erzeugen, wobei jedes demodulierte Detektorsignal einer der Modulationsfrequenzen entspricht,

wobei das Analysegerät (114) ferner mindestens ein Fehlererfassungsgerät (144) umfasst, das zum Durchführen einer Fehlererfassung basierend auf einem Vergleich der mindestens zwei demodulierten Detektorsignale eingerichtet ist, **dadurch gekennzeichnet, dass** die mindestens eine Lichtquelle (127) mindestens eine erste Lichtquelle (128) umfasst, die dazu ausgelegt ist, von dem mindestens einen Modulationsgerät (124) mit mindestens zwei verschiedenen Modulationsfrequenzen moduliert zu werden, und mindestens eine zweite Lichtquelle (130), die dazu ausgelegt ist, von dem mindestens einen Modulationsgerät (124) mit mindestens zwei verschiedenen Modulationsfrequenzen moduliert zu werden, die sich von den mindestens zwei verschiedenen Modulationsfrequenzen unterscheiden, mit denen die erste Lichtquelle (128) moduliert wird.

**17.** Analysesystem (110) zur Bestimmung einer Konzentration von mindestens einem Analyt in einer Körperflüssigkeit (112), wobei das Analysesystem (110) das Analysegerät (114) nach dem vorhergehenden Anspruch umfasst, wobei das Analysesystem (110) ferner mindestens einen Testträger (116) umfasst.

**Revendications**

**1.** Procédé pour déterminer une concentration d'au moins un substance à analyser dans un fluide corporel (112), le procédé comprenant :

a) application d'un échantillon du fluide corporel (112) sur un support de test (116) ;
b) éclairage du support de test (116) en utilisant au moins une source de lumière (127) ;
c) réception de la lumière renvoyée par le support de test (116) en utilisant au moins un détecteur (132) ;
d) détermination de la concentration de la substance à analyser en évaluant au moins un signal de détecteur (133) généré par le détecteur (132) ;

dans lequel l'au moins une source de lumière (127) est modulé en utilisant au moins deux fréquences de modulation différentes, dans lequel le signal de détecteur (133) est démodulé avec les au moins deux fréquences de modulation différentes afin de générer au moins deux signaux de détecteur démodulés, chaque signal de détecteur démodulé correspondant à l'une des fréquences de modulation, dans lequel le procédé comprend une détection de défaut basée sur une comparaison des au moins deux signaux de détecteur démodulés, **caractérisé en ce que** l'au moins une source de lumière (127) comprend au moins une première source de lumière (128) modulée par au moins deux fréquences de modulation différentes et au moins une seconde source de lumière (130) modulée par au moins deux fréquences de modulation différentes étant différentes des au moins deux fréquences de modulation différentes par lesquelles la première source de lumière (128) est modulée.

**2.** Procédé selon la revendication précédente, dans lequel la détection de défaut est une détection de défaut en ligne qui est effectuée de manière permanente ou répétée.

**3.** Procédé selon l'une quelconque des revendications précédentes, dans lequel l'au moins une source de lumière (127) est modulé en utilisant au moins trois fréquences de modulation différentes.

**4.** Procédé selon l'une quelconque des revendications précédentes, dans lequel la détection de défaut comprend la détection de signaux de détecteur démodulés défectueux.

**5.** Procédé selon la revendication précédente, dans lequel la détection de défaut comprend en outre le rejet des signaux de détecteur démodulés défectueux et l'utilisation de signaux de détecteur démodulés non défectueux uniquement pour déterminer la concentration de l'au moins un substance à analyser dans le fluide corporel (112).

**6.** Procédé selon la revendication précédente, dans lequel au moins un signal de détecteur démodulé défectueux est utilisé pour déterminer la concentration de la substance à analyser, dans lequel le degré de propension au défaut est pris en compte.

**7.** Procédé selon l'une quelconque des revendications précédentes, dans lequel au moins deux signaux de détecteur démodulés sont générés pour les fréquences de modulation par lesquelles la première source de lumière (128) est modulée et dans lequel au moins deux signaux de détecteur démodulés sont générés pour les fréquences de modulation par lesquelles la seconde source de lumière (130) est modulée.

8. Procédé selon la revendication précédente, dans lequel la détection du défaut est effectuée à la fois pour les signaux de détecteur démodulés aux fréquences de modulation par lesquelles la première source de lumière (128) est modulée et pour les signaux de détecteur démodulés aux les fréquences de modulation par lesquelles la seconde source de lumière (130) est modulée.

9. Procédé selon l'une quelconque des revendications précédentes, dans lequel la détection du défaut est effectuée au moins une fois avant d'appliquer l'échantillon du fluide corporel (112) sur le support de test (116).

10. Procédé selon la revendication précédente, dans lequel le procédé comprend en outre la détermination d'au moins une valeur à vide et à sec en évaluant l'au moins un signal de détecteur généré par le détecteur (132) avant d'appliquer l'échantillon du fluide corporel (112) au support de test (116).

11. Procédé selon l'une quelconque des revendications précédentes, dans lequel le procédé comprend en outre au moins une étape de vérification de la position, dans lequel l'étape de vérification de la position comprend les étapes du procédé suivantes :

    i) introduction du support de test (116) dans un dispositif d'analyse (114) ;
    ii) éclairage du support de test (116) par la source ou les sources de lumière (127) ;
    iii) réception de la lumière renvoyée par le support de test (116) en utilisant l'au moins un détecteur (132) ;
    iv) détermination d'au moins une position du support de test (116) dans le dispositif d'analyse (114) en évaluant au moins un signal de détecteur (133) généré par le détecteur (132), dans lequel la position comprend au moins l'un parmi un emplacement et/ou une orientation du support de test (116).

12. Procédé selon l'une quelconque des revendications précédentes, dans lequel le procédé comprend en outre au moins une étape de détection de défaut à la lumière ambiante, dans lequel l'étape de détection de défaut à la lumière ambiante comprend les étapes du procédé suivantes :

    I. réception de la lumière ambiante en utilisant l'au moins un détecteur (132) ;

    II. évaluation d'au moins un signal de détecteur (133) généré par le détecteur (132) ;
    III. effectuer une détection de défaut de lumière ambiante en comparant le signal ou les signaux de détecteur (133) générés par le détecteur (132) aux fréquences de modulation.

13. Procédé selon l'une quelconque des revendications précédentes, dans lequel la démodulation comprend la multiplication de façon indépendante du signal de détecteur (133) par les fréquences de modulation et le filtrage des résultats en utilisant des filtres passe-bas (138).

14. Procédé selon la revendication précédente, dans lequel la démodulation, avant de multiplier le signal de détecteur (133) par les fréquences de modulation, comprend le filtrage du signal de détecteur (133) en utilisant au moins un filtre passe-bande (142).

15. Procédé selon l'une quelconque des revendications précédentes, dans lequel le procédé comprend en outre au moins l'une des étapes de procédé suivantes :

    - introduction du support de test (116) dans un dispositif d'analyse (114) ;
    - lancement de la détection du défaut ;
    - acquisition d'une valeur à vide et à sec.

16. Dispositif d'analyse (114) pour déterminer une concentration d'au moins un substance à analyser dans un fluide corporel (112), le dispositif d'analyse (114) comprenant au moins un réceptacle (122) pour recevoir au moins un support de test (116), dans lequel au moins un échantillon du fluide corporel (112) est applicable au support de test (116), le dispositif d'analyse (114) comprenant en outre au moins une source de lumière (127) adaptée pour éclairer le support de test (116), le dispositif d'analyse (114) comprenant en outre au moins un détecteur (132) adapté pour recevoir lumière renvoyée par le support de test (116), le dispositif d'analyse (114) comprenant en outre au moins une unité d'évaluation (148) adaptée pour déterminer la concentration de la substance à analyser en évaluant au moins un signal de détecteur (133) généré par le détecteur (132), le dispositif d'analyse (114) comprenant en outre au moins un dispositif de modulation (124) adapté pour moduler la source de lumière (127) en utilisant au moins deux fréquences de modulation différentes, le dispositif d'analyse (114) comprenant en outre au moins un dispositif de démodulation (134) adapté pour démoduler le signal de détecteur (133) par les au moins deux fréquences de modulation différentes afin de générer au moins deux signaux de détecteur démodulés, chaque signal de détecteur démodulé correspondant à l'une des fréquences de modulation,

le dispositif d'analyse (114) comprenant en outre au moins un dispositif de détection de défaut (144) adapté pour effectuer une détection de défaut sur la base d'une comparaison des au moins deux signaux de détecteur démodulés, **caractérisé en ce que** la ou les sources de lumière (127) comprennent au moins une première source de lumière (128) configurée pour être modulée par ledit ou lesdits dispositifs de modulation (124) par au moins deux fréquences de modulation différentes et au moins une seconde source de lumière (130) configurée pour être modulée par ledit au moins un dispositif de modulation (124) par au moins deux fréquences de modulation différentes, étant différentes des au moins deux fréquences de modulation différentes par lesquelles la première source de lumière (128) est modulée.

17. Système d'analyse (110) pour déterminer une concentration d'au moins une substance à analyser dans un fluide corporel (112), le système d'analyse (110) comprenant le dispositif d'analyse (114) selon la revendication précédente, le système d'analyse (110) comprenant en outre au moins un support de test (116).

Fig. 1

val ($f_{1a}$) $\overset{?}{=}$ val ($f_{1b}$)

val ($f_{1a}$) $\overset{?}{=}$ val ($f_{1c}$)

val ($f_{1b}$) $\overset{?}{=}$ val ($f_{1c}$)

Error

val ($f_{2a}$) $\overset{?}{=}$ val ($f_{2b}$)

val ($f_{2a}$) $\overset{?}{=}$ val ($f_{2c}$)

val ($f_{2b}$) $\overset{?}{=}$ val ($f_{2c}$)

Error

$f_{1a,b,c}$

$f_{2a,b,c}$

EP 3 074 758 B1

31

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0632262 B1
- EP 1912058 B1
- US 2003169426 A1
- US 5526120 A
- WO 2010094426 A1
- WO 2010094427 A1
- WO 2007012494 A1
- WO 2009103540 A1
- WO 2011012269 A2
- WO 2011012270 A1
- WO 2011012271 A2
- EP 0354441 A2
- EP 0431456 A1
- EP 0302287 A2
- EP 0547710 A2
- EP 1593434 A2
- EP 0821234 A
- US 20020146835 A1
- EP 0821234 B1
- WO 2011061257 A1
- US 20080087819 A1
- WO 0125760 A1
- EP 1413883 A1
- WO 2006138226 A2

### Non-patent literature cited in the description

- **J. HONES et al.** The Technology Behind Glucose Meters: Test Strips. *Diabetes Technology & Therapeutics,* 2008, vol. 10 (1), 10-26
- **J. HOENES et al.** *The Technology Behind Glucose Meters: Test Strips, Diabetes Technology & Therapeutics,* 2008, vol. 10 (1), 10-26